# EUROPEAN PATENT APPLICATION

(11) **EP 4 023 235 A1**
(43) Date of publication of application: **06.07.2022**
(21) Application number: 20859414.3
(22) Date of filing: 31.08.2020
(51) Int. Cl.: A61K 35/768, A61K 31/17, A61K 31/454, A61K 31/4985, A61K 31/519, A61K 45/06, A61P 35/00

(54) **PHARMACEUTICAL COMPOSITION FOR TREATING CANCER, COMPRISING VACCINIA VIRUS AND GRANULOPOIESIS INHIBITOR AS ACTIVE INGREDIENTS**

(30) Priority: 29.08.2019 KR 20190106736
(71) Applicant: Bionoxx Inc., Seongnam-si, Gyeonggi-do 13554 (KR)
(72) Inventor: HWANG, Tae-Ho, Yangsan-si, Gyeongsangnam-do 50613 (KR); CHO, Mong, Yangsan-si, Gyeongsangnam-do 50613 (KR); KIM, Jae-Joon, Yangsan-si, Gyeongsangnam-do 50658 (KR)
(74) Representative: HGF
(86) International application number: PCT/KR2020/011648
(87) International publication number: WO 2021/040496

(57) **Abstract**

The present invention relates to a pharmaceutical composition for preventing or treating cancer, comprising a vaccinia virus and a granulopoiesis inhibitor as active ingredients. The pharmaceutical composition for treating cancer, comprising a vaccinia virus and a granulopoiesis inhibitor as active ingredients, of the present invention has a excellent anticancer effect and safety compared to the case of administering only the vaccinia virus. Therefore, the pharmaceutical composition comprising a vaccinia virus and a granulopoiesis inhibitor as active ingredients of the present invention may be efficiently utilized in treating cancer.

<110>    Bionoxx Inc.

<120>    PHARMACEUTICAL COMPOSITION FOR TREATING CANCER COMPRISING
         VACCINIA VIRUS AND NEUTROPHIL INHIBITOR

<130>    PCC008061BNX

<150>    KR 10-2019-0106736
<151>    2019-08-29

<160>    2

<170>    KoPatentIn 3.0

<210>    1
<211>    993
<212>    DNA
<213>    Artificial Sequence

<220>
<223>    nucleotide sequence for 330 aa fregment of HSV-TK (OTS-412)

<400>    1
ttagtcgtaa tccaggataa agacgtgcat gggacggagg cgtttggcca agacgtccaa        60

ggcccaggca aacacgttat acaggtcgcc gttgggggcc agcaactcgg gggcccgaaa       120

cagggtaaat aacgtgtccc cgatatgggg tcgtgggccc gcgttgctct ggggctcggc       180

accctggggc ggcacggccg tccccgaaag ctgtccccaa tcctcccacc acgacccgcc       240

gccctgcaga taccgcaccg tattggcaag cagcccgtaa acgcggcgaa tcgcggccag       300

catagccagg tcaagccgct cgccggggcg ctggcgtttg gccaggcggt cgatgtgtct       360

gtcctccgga agggccccca acacgatgtt tgtgccgggc aaggtcggcg ggatgagggc       420

cacgaacgcc agcacggcct ggggggtcat gctgcccata aggtatcgcg cggccgggta       480

gcacaggagg gcggcgatgg gatggcggtc gaagatgagg gtgagggccg ggggcggggc       540

atgtgaactc ccagcctccc ccccgacatg aggagccaga acggcgtcgg tcacggcata       600

aggcatgccc attgttatct gggcgcttgt cattaccacc gccgcgtccc cggccgatat       660

ctcaccctgg tcgaggcggt gttgtgtggt gtagatgttc gcgattgtct cggaagcccc       720

cagcacctgc cagtaagtca tcggctcggg tacgtagacg atatcgtcgc gcgaacccag       780

ggccaccagc agttgcgtgg tggtggtttt ccccatcccg tgaggaccgt ctatataaac       840

ccgcagtagc gtgggcattt tctgctccag gcggacttcc gtggcttctt gctgccggcg       900

agggcgcaac gccgtacgtc ggttgctatg gccgcgagaa cgcgcagcct ggtcgaacgc       960

agacgcgtgt tgatggcagg ggtacgaagc cat                                    993

<210>    2
<211>    1131

<212>	DNA
<213>	Artificial Sequence

<220>
<223>	nucleotide sequence for mutated HSV-TK of WOTS-418

<400>	2
atggcttcgt acccctgcca tcaacacgcg tctgcgttcg accaggctgc gcgttctcgc	60

ggccatagca accgacgtac ggcgttgcgc cctcgccggc agcaagaagc cacggaagtc	120

cgcctggagc agaaaatgcc cacgctactg cgggtttata tagacggtcc tcacgggatg	180

gggaaaacca ccaccacgca actgctggtg gccctgggtt cgcgcgacga tatcgtctac	240

gtacccgagc cgatgactta ctggcaggtg ctgggggctt ccgagacaat cgcgaacatc	300

tacaccacac aacaccgcct cgaccagggt gagatatcgg ccggggacgc ggcggtggta	360

atgacaagcg cccagataac aatgggcatg ccttatgccg tgaccgacgc cgttctggct	420

cctcatgtcg gtggtgaggc tgggagttca catgccccgc ccccggccct caccctcatc	480

ttcgaccgcc atcccatcta cgccctcctg tgctacccgg ccgcgcgata ccttatgggc	540

agcatgaccc ctcaggccgt gctggcgttc gtggccctca tcccgccgac cttgcccggc	600

acaaacatcg tgttgggggc ccttccggag gacagacaca tcgaccgcct ggccaaacgc	660

cagcgccccg gcgagcggct tgacctggct atgctggccg cgattcgccg cgtttacggg	720

ctgcttgcca atacggtgcg gtatctgcag ggcggcgggt cgtggtggga ggattgggga	780

cagctttcgg ggacggccgt gccgccccag ggtgccgagc cccagagcaa cgcgggccca	840

cgaccccata tcggggacac gttatttacc ctgtttcggg cccccgagtt gctggccccc	900

aacggcgacc tgtataacgt gtttgcctgg gccttggacg tcttggccaa acgcctccgt	960

cccatgcacg tctttatcct ggattacgac caatcgcccg ccggctgccg ggacgccctg	1020

ctgcaactta cctccgggat ggtccagacc cacgtcacca ccccaggctc cataccgacg	1080

atctgcgacc tggcgcgcac gtttgcccgg gagatggggg aggctaacta a	1131

## Description

### Technical Field

The present invention relates to a pharmaceutical composition for treating cancer, comprising, as active ingredients, a vaccinia virus and a granulopoiesis inhibitor.

### Background Art

Oncolytic viruses have excellent tumor-specific targeting ability, proliferation ability in cancer cells, and cancer cell-killing ability. Recently, various clinical studies based on oncolytic viruses have been conducted. In the year 2015, an era of oncolytic virus field began in the US and Europe, as talimogene laherparepvec (T-Vec), which is an oncolytic virus based on herpes simplex virus, was successfully commercialized as a therapeutic agent for advanced melanoma.

Recently, the usefulness of oncolytic viruses exceeds their own efficacy and the viruses activate tumor immunity, thereby showing their potential as a therapeutic agent that is used in combination with another immunotherapeutic agent. Until the year 2000 that was an early stage of development of oncolytic viruses, a direct killing effect of the viruses, which is caused by cancer cell-specific proliferation thereof, was relatively more important. However, subsequent clinical studies have found that activation of tumor immunity is a key mechanism rather than a direct cancer cell-killing effect. Based on this finding, therapeutic agents which include an oncolytic virus and an immunotherapeutic agent such as an immune checkpoint inhibitor, both being administered in combination, are recently being developed. This is because it is known that oncolytic viruses convert the tumor microenvironment, in which immunity is suppressed, into a tumor microenvironment appropriate for immunotherapy.

In a number of clinical studies on vaccinia virus-based oncolytic viruses, oncolytic virus therapy may result in acute tumor necrosis, durable response, or complete response, but in some cases, may lead to a difficult-to-predict result (pharmacodynamics variability) such as progressive disease or early death. For example, for Pexa-vec that is based on a vaccinia virus, in the phase 1 clinical trial, some patients died prematurely within a month after the oncolytic virus therapy and this was associated with persistent systemic inflammatory response and main organs dysfunction. In addition, transient flu symptoms (high fever) and low blood pressure observed after oncolytic virus treatment are the most frequent adverse events following the oncolytic virus therapy.

Meanwhile, in the treatment using oncolytic viruses, there has been no accurate report on the effect of a drug-induced increase in neutrophils on the treatment result. The first innate immune cell that responds to oncolytic virus administration is a neutrophil, which has a short half-life of less than 20 hours in the human body. Clinically, although a high number of neutrophils were observed in patients treated with drugs (*e.g.,* clozapine) or with acute inflammation and acute injury (Liao Y et al, PloS One, 8(7), 2013), the increase of the absolute neutrophil count (ANC) is not recognized as an abnormal response because this is not included in the Common Terminology Criteria of Adverse Events (CTCAE).

Therefore, there is a need for the study of the effect of changes in the number of neutrophils on oncolytic virus treatment.

### Disclosure of Invention

### Technical Problem

Accordingly, as a result of conducting studies to enhance the anticancer effect of a vaccinia virus used as an oncolytic virus, the present inventors have found that in administering a vaccinia virus to an individual having cancer, when an inhibitor that lowers neutrophil levels is administered in combination, the co-administration could significantly reduce the systemic inflammatory response to ensure safe use, as compared with the existing case where a vaccinia virus is administered alone. Additionally, the present inventors have found that when the inhibitor is administered in combination, the cancer cell-specific selectivity and proliferative capacity of the vaccinia virus are improved, thereby completing the present invention. It is speculated that the inhibitor inhibits the granulopoiesis, thereby lowering the neutrophil level, and thus improving the anticancer effect of the oncolytic virus.

### Solution to Problem

To achieve the above-mentioned object, in an aspect of the present invention, there is provided a pharmaceutical composition for treating cancer, comprising, as active ingredients, a vaccinia virus and granulopoiesis inhibitor.

In another aspect of the present invention, there is provided a method for treating cancer, comprising administering, to an individual having cancer, a vaccinia virus and granulopoiesis inhibitor.

In yet another aspect of the present invention, there is provided a use of a composition including a vaccinia virus and granulopoiesis inhibitor, for the prevention or treatment of cancer.

In still yet another aspect of the present invention, there is provided a use of a composition including a vaccinia virus and granulopoiesis inhibitor, for the manufacture of a medicament for preventing or treating cancer.

In still yet another aspect of the present invention, there is provided an anticancer adjuvant, comprising granulopoiesis inhibitor as an active ingredient.

### Advantageous Effects of Invention

The pharmaceutical composition for treating cancer, which comprises, as active ingredients, a vaccinia virus and granulopoiesis inhibitor, of the present invention has excellent anticancer effect and safety as compared with a conventional case where only a vaccinia virus is administered. Accordingly, the pharmaceutical composition, which comprises, as active ingredients, a vaccinia virus and granulopoiesis inhibitor, of the present invention may be effectively used for the treatment of cancer.

### Brief Description of Drawings

FIG. 1 illustrates results obtained by administering, to mouse renal cancer cell-transplanted mice (Renca), a wild-type vaccinia virus (Western Reserve strain vaccinia virus, WR) and hydroxyurea (HU), and then measuring tumor volumes on days 0, 3, 7, 10, and 14.
FIG. 2 illustrates results obtained by administering, to the mouse renal cancer cell-transplanted mice (Renca), the wild-type vaccinia virus (WR) and HU, and then measuring body weights on days 0, 3, 7, 10, and 14.
FIG. 3 illustrates results obtained by administering, to mouse renal cancer cell-transplanted mice (Renca), a recombinant vaccinia virus (WR VV^{tk-}), which has been obtained by deleting TK gene from WR, and HU (60 mg/kg), and then measuring tumor volumes on days 0, 3, 7, 10, 14, 17, and 21.
FIG. 4 illustrates results obtained by administering, to mouse renal cancer cell-transplanted mice (Renca), the recombinant vaccinia virus (WR VV^{tk-}) and HU (30 mg/kg), and then measuring tumor volumes on days 0, 3, 7, 10, and 14.
FIG. 5 illustrates results obtained by measuring tumor volumes 1 day before and on days 4 and 7 after administering, to mouse melanoma-transplanted mice (B16F10), a recombinant vaccinia virus (VV DD), which has been obtained by simultaneously deleting TK gene and vaccinia virus growth factor (VGF) gene from WR, and HU.
FIG. 6 illustrates results obtained by administering, to human lung cancer cell (NCI-H460)-transplanted mice, a recombinant vaccinia virus (WOTS-418) and HU, and then measuring tumor volumes on days 0, 5, 10, 12, and 15.
FIG. 7 illustrates results obtained by administering, to human lung cancer cell (NCI-H460)-transplanted mice, the recombinant vaccinia virus (WOTS-418) and HU, and then measuring survival rates.
FIG. 8 illustrates results obtained by administering, to mouse renal cancer cell-transplanted mice (Renca), a recombinant vaccinia virus (VV^{tk-}) and human granulocyte colony stimulating factor (rhG-CSF) or HU, and then measuring tumor volumes in the mice.
FIG. 9 illustrates results obtained by isolating lymphocytes in the spleen from the mouse renal cancer cell-transplanted mice (Renca), to which the recombinant vaccinia virus (VV^{tk-}) and the human granulocyte colony stimulating factor (rhG-CSF) or HU have been administered, administering the lymphocytes to new mice, and then measuring tumor volumes in the new mice.
FIG. 10 illustrates results obtained by administering, to mouse renal cancer cell-transplanted mice (Renca), a recombinant vaccinia virus (Wyeth VV^{tk-}) and HU, and then measuring tumor volumes in the mice.
FIG. 11 illustrates results obtained by isolating T lymphocytes from mouse renal cancer cell-transplanted mice (Renca), to which a recombinant vaccinia virus (Wyeth VV^{tk-}) and HU have been administered, administering the T lymphocytes to new mice, and then measuring tumor volumes in the new mice.
FIG. 12 illustrates results obtained by isolating splenocytes isolated from the mouse renal cancer cell-transplanted mice (Renca), to which the recombinant vaccinia virus (Wyeth VV^{tk-}) and HU have been administered, administering the splenocytes to new mice, and then measuring tumor volumes in the new mice.
FIG. 13 illustrates results obtained by administering, to mouse renal cancer cell-transplanted mice (Renca), a recombinant vaccinia virus (Wyeth VV^{tk}) and HU, and then measuring tumor volumes on day 22.
FIG. 14 illustrates results obtained by administering, to mouse renal cancer cell-transplanted mice (Renca), a recombinant vaccinia virus (Wyeth VV^{tk}) and HU, and then observing the proliferation of CD4+ T cells or CD8+ T cells in the spleen tissue.
FIG. 15 illustrates results obtained by administering, to mouse breast cancer cell-transplanted mice (4T1), a recombinant vaccinia virus (OTS-412) and HU, and then observing the proliferation of CD4+ T cells or CD8+ T cells in the blood and spleen tissue.
FIG. 16 illustrates results obtained by administering, to the left tumor in mouse breast cancer cell-transplanted mice (4T1), a recombinant vaccinia virus (WR VV^{tk-}) and HU, and then measuring left tumor volumes.
FIG. 17 illustrates results obtained by administering, to the left tumor in mouse breast cancer cell-transplanted mice (4T1), a recombinant vaccinia virus (WR VV^{tk-}) and HU, and then measuring right tumor volumes.
FIG. 18 illustrates results obtained by administering, to mouse renal cancer cell-transplanted mice (Renca), a recombinant vaccinia virus (WR) and HU, and then performing staining on day 22 to identify distribution of the recombinant vaccinia virus in mouse tumor tissues.
FIG. 19 illustrates results obtained by administering, to normal mice, a wild-type vaccinia virus (WR) or a wild-type vaccinia virus (WR) and HU, and then identifying distribution of the wild-type vaccinia virus in liver and kidney tissues.
FIG. 20 illustrates the absolute neutrophil count of mice in each group after administering, to mouse renal cancer cell-transplanted mice (Renca), saline, HU, a recombinant vaccinia virus (OTS-412), a recombinant vaccinia virus and a recombinant human granulocyte colony-stimulating factor (OTS-412+rhG-CSF), or a recombinant vaccinia virus and HU (OTS-412+HU).
FIG. 21 illustrates the blood neutrophil count of mice measured in each group after administering, to mouse renal cancer cell-transplanted mice (Renca), saline, a recombinant vaccinia virus, or a recombinant vaccinia virus (WR VV^{tk-}) and HU.
FIG. 22 illustrates the blood neutrophil count of mice measured in each group after administering, to mouse renal cancer cell-transplanted mice (Renca), saline, a recombinant vaccinia virus, or a recombinant vaccinia virus (WOTS-418) and HU.
FIG. 23 illustrates the blood neutrophil count of mice measured in each group after administering, to mouse renal cancer cell-transplanted mice (Renca), saline, lenalidomide, or HU.
FIG. 24 illustrates the blood neutrophil count of mice measured in each group after administering, to mouse renal cancer cell-transplanted mice (Renca), saline, a recombinant vaccinia virus, a recombinant vaccinia virus (WOTS-418) and lenalidomide, or a recombinant vaccinia virus (WOTS-418) and HU.
FIG. 25 illustrates the tumor volume of mice measured after administering, to mouse renal cancer cell-transplanted mice (Renca), a recombinant vaccinia virus (WR VV^{tk-}) and lenalidomide.
FIG. 26 illustrates the tumor volume of mice measured after administering, to mouse renal cancer cell-transplanted mice (Renca), a recombinant vaccinia virus (WR VV^{tk-}) and palbociclib.
FIG. 27 illustrates the body weight of mice measured after administering, to mouse renal cancer cell-transplanted mice (Renca), a recombinant vaccinia virus (WR VV^{tk-}) and palbociclib.
FIG. 28 illustrates the tumor volume of mice measured on day 0, day 4, day 10, day 14, day 17, and day 21 after administering, to mouse renal cancer cell-transplanted mice (Renca), an oncolytic virus (Wyeth VV^{tk-}), a PD-1 inhibitor, and HU.
FIG. 29 illustrates the tumor volume of mice measured on day 0, day 4, day 10, day 14, and day 17 after administering, to mouse renal cancer cell-transplanted mice (Renca), an oncolytic virus (Wyeth VV^{tk-}), a CTLA-4 inhibitor, and HU.
FIG. 30 illustrates the tumor volume of mice measured on day 0, day 4, day 10, day 14, day 17, and day 21 after administering, to mouse renal cancer cell-transplanted mice (Renca), an oncolytic virus (Wyeth VV^{tk-}), a PD-L1 inhibitor, and HU.
FIG. 31 illustrates the tumor volume of mice measured on day 0, day 3, day 7, day 10, and day 14 after administering, to mouse breast cancer cell-transplanted mice (4T1), an oncolytic virus (WR VV^{tk-}), a CTLA-4 inhibitor, and HU.
FIG. 32 illustrates the tumor volume of mice measured on day 0, day 3, day 7, day 10, day 14, and day 18 after administering, to mouse breast cancer cell-transplanted mice (4T1), an oncolytic virus (WOTS-418), a PD-L1 inhibitor, and HU.
FIG. 33 illustrates the tumor volume of mice measured on day 0, day 3, and day 7 after administering, to mouse renal cancer cell-transplanted mice (Renca), a Western Reserve strain vaccinia virus (WR), a CTLA-4 inhibitor, and HU.

### Best Mode for Carrying out the Invention

Hereinafter, the present invention will be specifically described.

In an aspect of the present invention, there is provided a pharmaceutical composition for preventing or treating cancer, comprising, as active ingredients, a vaccinia virus and granulopoiesis inhibitor.

The vaccinia virus and granulopoiesis inhibitor contained in the pharmaceutical composition may be administered in combination simultaneously, sequentially, or in reverse order. Specifically, the vaccinia virus and granulopoiesis inhibitor may be administered simultaneously. In addition, the granulopoiesis inhibitor may be first administered, followed by the vaccinia virus. Furthermore, the vaccinia virus may be first administered, followed by the granulopoiesis inhibitor. In addition, the granulopoiesis inhibitor may be first administered, followed by the vaccinia virus, and the granulopoiesis inhibitor may be administered again.

The vaccinia virus may belong to, but is not limited to, Western Reserve (WR), New York vaccinia virus (NYVAC), Wyeth (The New York City Board of Health; NYCBOH), LC16m8, Lister, Copenhagen, Tian Tan, USSR, Tashkent, Evans, International Health Division-J (IHD-J), or International Health Division-White (IHD-W) vaccinia virus strain. In an embodiment of the present invention, Western Reserve strain vaccinia virus and Wyeth strain vaccinia virus were used.

The vaccinia virus may be a wild-type vaccinia virus or a recombinant vaccinia virus. Specifically, the recombinant vaccinia virus may be obtained by deleting a gene from a wild-type vaccinia virus or inserting a foreign gene thereinto. Here, among the genes of the wild-type vaccinia virus, a gene related to viral virulence may be deleted which encodes any one selected from the group consisting of thymidine kinase (TK), vaccinia growth factor (VGF), WR53.5, F13.5L, F14.5L, A56R, B18R, or combinations thereof.

In addition, the inserted foreign gene may be a gene that promotes immunity and encodes any one selected from the group consisting of herpes simplex virus thymidine kinase (HSV-TK), mutated HSV-TK, granulocyte-macrophage colony-stimulating factor (GM-CSF), granulocyte colony-stimulating factor (G-CSF), cytosine deaminase (CD), carboxyl esterase type 1, carboxyl esterase type 2, interferon beta (INF-β), somatostatin receptor 2, and combinations thereof.

Specifically, the recombinant vaccinia virus may be obtained by deleting TK gene from a vaccinia virus that belongs to Western Reserve (WR), New York vaccinia virus (NYVAC), Wyeth (The New York City Board of Health; NYCBOH), LC16m8, Lister, Copenhagen, Tian Tan, USSR, Tashkent, Evans, International Health Division-J (IHD-J), or International Health Division-White (IHD-W) vaccinia virus strain. In an embodiment of the present invention, a recombinant vaccinia virus obtained by deleting TK gene from a Western Reserve strain vaccinia virus was used, and this virus was designated "WR VV^{tk-}". In addition, in an embodiment of the present invention, a recombinant vaccinia virus obtained by deleting TK gene from a Wyeth strain vaccinia virus was used, and this virus was designated "Wyeth VV^{tk-}".

In addition, the recombinant vaccinia virus may be obtained by deleting TK gene and VGF gene from a vaccinia virus that belongs to Western Reserve, NYVAC, Wyeth, LC16m8, Lister, Copenhagen, Tian Tan, USSR, Tashkent, Evans, IHD-J, or IHD-W vaccinia virus strain. In an embodiment of the present invention, a recombinant vaccinia virus obtained by deleting TK gene and VGF gene from a Western Reserve strain vaccinia virus was used, and this virus was designated "VV DD".

Furthermore, the recombinant vaccinia virus may be obtained by deleting TK gene from and inserting HSV-TK gene into a vaccinia virus that belongs to Western Reserve, NYVAC, Wyeth, LC16m8, Lister, Copenhagen, Tian Tan, USSR, Tashkent, Evans, IHD-J, or IHD-W vaccinia virus strain.

In addition, the recombinant vaccinia virus may be obtained by deleting TK gene from and inserting mutated HSV-TK gene into a vaccinia virus that belongs to Western Reserve, NYVAC, Wyeth, LC16m8, Lister, Copenhagen, Tian Tan, USSR, Tashkent, Evans, IHD-J, or IHD-W vaccinia virus strain. In an embodiment of the present invention, a recombinant vaccinia virus obtained by deleting TK gene from a Wyeth strain vaccinia virus and inserting, into the deleted position, a gene encoding the HSV-TK fragment (1-330 aa) of SEQ ID NO: 1 was used, and this virus was designated "OTS-412". In addition, in an embodiment of the present invention, a recombinant vaccinia virus obtained by deleting TK gene from a Western Reserve strain vaccinia virus and inserting, into the deleted position, a gene encoding the HSV-TK variant of SEQ ID NO: 2 of HSV-TK gene was used, and this virus was designated "WOTS-418".

Furthermore, the recombinant vaccinia virus may be obtained by deleting TK gene from and inserting GM-CSF gene into a vaccinia virus that belongs to Western Reserve, NYVAC, Wyeth, LC16m8, Lister, Copenhagen, Tian Tan, USSR, Tashkent, Evans, IHD-J, or IHD-W vaccinia virus strain.

In addition, the recombinant vaccinia virus may be obtained by deleting TK gene from and inserting G-CSF gene into a vaccinia virus that belongs to Western Reserve, NYVAC, Wyeth, LC16m8, Lister, Copenhagen, Tian Tan, USSR, Tashkent, Evans, IHD-J, or IHD-W vaccinia virus strain.

Furthermore, the recombinant vaccinia virus may be obtained by deleting TK gene from and inserting cytosine deaminase (CD) gene into a vaccinia virus that belongs to Western Reserve, NYVAC, Wyeth, LC16m8, Lister, Copenhagen, Tian Tan, USSR, Tashkent, Evans, IHD-J, or IHD-W vaccinia virus strain.

In addition, the recombinant vaccinia virus may be obtained by deleting TK gene from and inserting somatostatin receptor 2 gene into a vaccinia virus that belongs to Western Reserve, NYVAC, Wyeth, LC16m8, Lister, Copenhagen, Tian Tan, USSR, Tashkent, Evans, IHD-J, or IHD-W vaccinia virus strain.

Furthermore, the recombinant vaccinia virus may be obtained by deleting TK gene from and inserting any two or more genes, which are selected from the group consisting of genes, each of which encodes herpes simplex virus thymidine kinase (HSV-TK), mutated HSV-TK, granulocyte-macrophage colony-stimulating factor (GM-CSF), granulocyte colony-stimulating factor (G-CSF), cytosine deaminase (CD), or somatostatin receptor 2, into a vaccinia virus that belongs to Western Reserve, NYVAC, Wyeth, LC16m8, Lister, Copenhagen, Tian Tan, USSR, Tashkent, Evans, IHD-J, or IHD-W vaccinia virus strain.

In addition, the recombinant vaccinia virus may be obtained by deleting TK gene and VGF gene from and inserting any one gene, which is selected from the group consisting of genes, each of which encodes herpes simplex virus thymidine kinase (HSV-TK), mutated HSV-TK, granulocyte-macrophage colony-stimulating factor (GM-CSF), granulocyte colony-stimulating factor (G-CSF), cytosine deaminase (CD), or somatostatin receptor 2, and combinations thereof, into a vaccinia virus that belongs to Western Reserve, NYVAC, Wyeth, LC16m8, Lister, Copenhagen, Tian Tan, USSR, Tashkent, Evans, IHD-J, or IHD-W vaccinia virus strain.

As used herein, the term "gene deletion" means that a gene is not expressed due to partial or complete deletion of the gene, or insertion of a foreign gene thereinto. In a case where partial deletion occurs in the gene, some amino acids at the N-terminus or C-terminus of a polypeptide expressed by the gene may be deleted.

As used herein, the term "thymidine kinase (TK)" refers to an enzyme that is called thymidine kinase and involved in nucleotide biosynthesis. TK is an enzyme used for nucleotide biosynthesis in both cells and viruses. Here, for the cells, normal cells do not divide anymore, and thus no TK exists therein; and even for rapidly dividing cells such as hair follicle cells, TK is not present in an amount sufficient for viruses to utilize. From these viewpoints, a virus is allowed to proliferate only in the presence of cancer cells, in which TK is present, by deletion of TK gene therein, so that the cancer cells may be selectively killed.

As used herein, the term "vaccinia growth factor (VGF)" refers to a polypeptide that has sequence homology to epidermal growth factor and stimulates cell proliferation around infected cells. A vaccinia virus replicates better in proliferating cells, and thus may be advantageously used for viral replication *in vivo.* In order to cause an oncolytic virus to proliferate more specifically only in cancer cells, the virus may additionally undergo deletion of VGF gene in addition to deletion of the TK gene.

As used herein, the term "GM-CSF", which is called granulocyte-macrophage colony-stimulating factor, refers to a protein secreted by macrophages, T cells, mast cells, natural killer cells, endothelial cells, and fibroblasts. GM-CSF stimulates stem cells to produce granulocytes (neutrophils, basophils, eosinophils) and monocytes. In addition, GM-CSF rapidly increases the number of macrophages, thereby inducing an immune response. GM-CSF may be of human origin and may be a protein having the sequence of GenBank: AAA52578.1.

As used herein, the term "CD", which is called cytosine deaminase, refers to an enzyme that catalyzes hydrolytic deamination of cytosine into uracil and ammonia.

As used herein, the term "G-CSF", which is called granulocyte colony-stimulating factor, refers to a cytokine produced by macrophages, fibroblasts, endothelial cells, and the like upon stimulation by inflammation or endotoxin. G-CSF promotes production of neutrophils. The G-CSF may be of human origin (rhGCSF) and may be a protein having the sequence of GenBank: AAA03056.1.

As used herein, the term "somatostatin receptor 2" refers to a protein encoded by SSTR2 gene in humans. The somatostatin receptor 2 is expressed mainly in tumors, and patients with neuroendocrine tumors, who overexpress somatostatin receptor 2, show improved prognosis. The somatostatin receptor 2 has capacity to stimulate apoptosis in many cells, including cancer cells.

A myeloid cell may be granulocytes, and specifically, the myeloid cells may be neutrophils, eosinophils, or basophils.

The granulopoiesis inhibitor may be a substance that inhibits granulocytes (*e*.*g*., neutrophils, eosinophils or basophils) mainly produced in bone marrow. The granulopoiesis inhibitor, when reducing or inhibiting the number of neutrophils (i.e., one of myeloid cells) in the body, may be referred to as a neutrophil inhibitor or include the same. The neutrophil is also called a neutrophilic leukocyte, and refers to a neutrophil cell circulating in the blood, which is a type of a granulocyte mainly produced in the bone marrow. Neutrophils are the main component of granulocytes, and the normal number is about 1,500 to about 8,000 per 1 mm³ of blood. The neutrophil absorbs, through phagocytosis, foreign substances such as bacteria that have invaded the body and breaks the foreign substances down with a digestive enzyme *(e.g.,* hydrogen peroxide, lysosome, *etc.).*

As used herein, the term "absolute neutrophil count (ANC)" refers to the number obtained by multiplication of the number of white blood cells × the percentage of neutrophils.

The granulopoiesis inhibitor may be hydroxyurea, lenalidomide, thalidomide, tadalafil, palbociclib, alkylating agents, anthracyclines, antimetabolites, camptothecins, epipodophyllotoxins, mitomycin C, taxanes, or vinblastine. The hydroxyurea may be a compound having the structure of Formula 1 below:

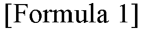

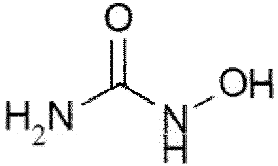

The hydroxyurea is known as an anticancer agent that inhibits DNA synthesis; however, the exact mechanism of action thereof is not elucidated. In addition, the hydroxyurea may be included in the pharmaceutical composition in the form of a commercialized drug that contains hydroxyurea. Examples of the commercialized drug that contains hydroxyurea may include, but are not limited to, Hydroxyurea^{®}, Hydrea^{®}, Droxia^{™}, Mylocel^{™}, Siklos^{®}, and Hydrine^{®} capsule. The hydroxyurea may be taken orally, and parenteral administration thereof is also possible.

The lenalidomide may be a compound having the structure of Formula 2 below:

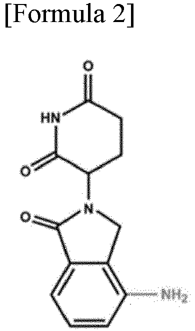

The lenalidomide is an anticancer agent used for the treatment of multiple myeloma, *etc.* In addition, the lenalidomide stops the growth cycle of cancer cells and inhibits cancer proliferation by activating tumor suppressor genes as an anticancer effect. As an immunomodulatory effect, the lenalidomide eliminates tumor cells by activating immune cells (*e.g.,* T cells, natural killer cells (NK cells), B cells, *etc.).* In addition, the lenalidomide has an angiogenesis inhibitory effect that inhibits the formation of new blood vessels to supply nutrients to cancer cells.

The thalidomide may be a compound having the structure of Formula 3 below:

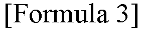

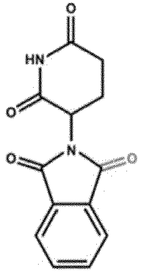

The exact mechanism of action of thalidomide is not known, but is used for the treatment of multiple myeloma and severe skin lesions in patients with leprosy (Hansen's disease).

The tadalafil may be a compound having the structure of Formula 4 below:

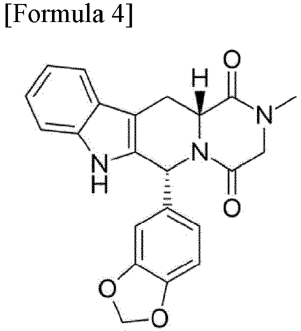

The palbociclib may be a compound having the structure of Formula 5 below:

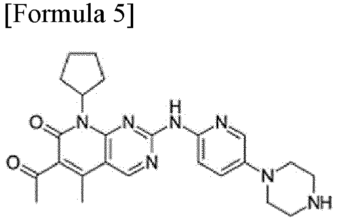

The alkylating agent may be nitrogen mustard, an ethylene derivative, an alkylsulfonic acid derivative, nitrosoureas, or triazines compounds among chemotherapeutic agents for malignant tumors. These may also be called in the name of an alkylating agent because they substitute the hydrogen in many organic compounds, proteins, or nucleic acids with an alkyl group. By alkylation with the alkylating agent, DNA replication and mRNA transcription of tumor cells may be inhibited and an antitumor action may be exhibited. As a common pharmacological action, the alkylating agent acts non-specifically on each phase of a cell cycle, and may inhibit cell division with a high proliferative potential. Since the alkylating agent exhibits a radiation-like action, dyshematopoiesis may be strong and immunosuppression may be caused.

The anthracycline is a drug extracted from *Streptomyces* bacteria, is used for cancer chemotherapy, and is used to treat many cancers including leukemia, lymphoma, breast cancer, gastric cancer, uterine cancer, ovarian cancer, bladder cancer, and lung cancer. The first discovered anthracycline was daunorubicin (trademark: Daunomycin), which was naturally produced by *Streptomyces peucetius,* which is a species of *Actinomycetes.* The most clinically important anthracyclines include doxorubicin, daunorubicin, epirubicin, idarubicin, *etc.*

The antimetabolite may be a substance that inhibits the development and proliferation of cells by antagonizing essential metabolites for the metabolism or growth of microorganisms or tumor cells. Slupamine, which is used as a chemotherapeutic agent and is antagonistic to the bacterial para-aminobenzoate (PABA), was historically first developed. The antimetabolites may include sulfa drugs for bacteria, purine antimetabolite drugs for malignant tumors (8-azaguanine, 6-thioguanine, 6-mercaptopurine), pyrimidine antimetabolite drugs (5-fluororuacil, cytarabine, azauridine), folate antimetabolite drugs (4-aminopterin, methotrexat), or glutamine antimetabolite drugs (azerin, DON).

The camptothecin may be a natural anticancer substance isolated from plants such as *Camptotheca acuminate* (Camptotheca, Happy tree), and *Chonemorpha fragrans.*

The camptothecin may be a compound having the structure of Formula 6 below:

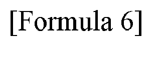

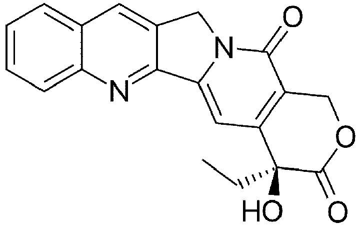

The epipodophyllotoxin may be a natural anticancer substance produced naturally in the root of *Podophyllum peltatum.* A derivative of epipodophyllotoxin may be used for cancer treatment at present.

The epipodophyllotoxin may be a compound having the structure of Formula 7 below:

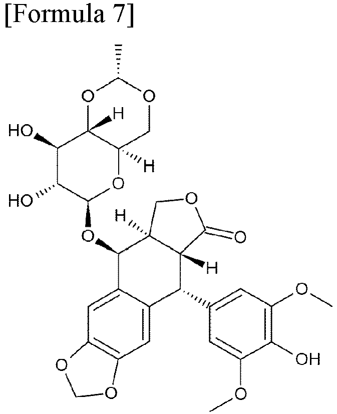

The mitomycin C may be an antibiotic substance isolated by *Streptomyces griseus.* The mitomycin C is thermally stable, has the lowest toxicity, and has a strong anticancer effect. The mitomycin C inhibits the cellular enzyme system and nucleic acid metabolism, thus inhibiting the division of cell nuclei, and thereby preventing the proliferation of malignant tumor cells. Examples of the side effects of mitomycin C include bleeding accompanied with leukopenia and thrombocytopenia, *etc.*

The taxane is also called a cell division inhibitor or anti-microtubule inhibitor, and may be an anticancer agent which inhibits cancer cell growth by inhibiting cell division. The taxane may kill cancer cells by disrupting the microtubules through which chromosomes move during cell mitosis. The taxane is used to treat various types of cancer such as breast cancer, ovarian cancer, and non-small cell lung cancer. Specifically, the taxane includes paclitaxel, docetaxel, *etc.*

The vinblastine may be an anticancer agent of a vinca alkaloid component used for the treatment of various types of cancer. The vinblastine was first extracted from the periwinkle plant belonging to the *Oleander* family, and a synthetic material is used at present. The vinblastine is the most widely used agent among anticancer agents and is also widely used in combination therapy with other anticancer agents. The vinblastine prevents the division of cancer cells by interfering with the normal function of microtubules. The vinblastine may widely be used for testicular cancer, breast cancer, lymphoma, Kaposi's sarcoma, *etc.* The most important side effect of vinblastine is a decrease in leukocytes and thrombocyte, and side effects such as gastrointestinal disorders, increased blood pressure, excessive sweating, depression, muscle pain, nausea, and headache may appear.

A dosage of the vaccinia virus varies depending on the individual's condition and body weight, the severity of disease, the type of drug, the route and period of administration, and may be appropriately selected by a person skilled in the art. The dosage may be such that a patient receives a vaccinia virus at 1×10⁵ to 1×10¹⁸ of virus particles, infectious virus units (TCID₅₀), or plaque forming units (pfu). Specifically, the dosage may be such that a patient receives a vaccinia virus at 1×10⁵, 2×10⁵, 5×10⁵, 1×10⁶, 2×10⁶, 5×10⁶, 1×10⁷, 2×10⁷, 5×10⁷, 1×10⁸, 2×10⁸, 5×10⁸, 1×10⁹, 2×10⁹, 5×10⁹, 1×10¹⁰, 5×10¹⁰, 1×10¹¹, 5×10¹¹, 1×10¹², 1×10¹³, 1×10¹⁴, 1×10¹⁵, 1×10¹⁶, 1×10¹⁷, or higher of virus particles, infectious virus units, or plaque forming units, and various numerical values and ranges between the above-mentioned numerical values may also be included therein. Preferably, the vaccinia virus may be administered at a dose of 1×10⁵ to 1×10¹⁰ pfu. More preferably, the vaccinia virus may be administered at a dose of equal to or greater than 1×10⁵ and lower than 1×10⁹ pfu. In an embodiment of the present invention, the vaccinia virus was administered at 1×10⁵ or 1×10⁷ pfu.

In addition, the granulopoiesis inhibitor may be administered at a dose of 1 mg/kg/day to 100 mg/kg/day, or 10 mg/kg/day to 90 mg/kg/day. Specifically, the granulopoiesis inhibitor may be administered at a dose of 10 mg/kg/day to 90 mg/kg/day, 15 mg/kg/day to 80 mg/kg/day, 20 mg/kg/day to 70 mg/kg/day, 25 mg/kg/day to 65 mg/kg/day, or 30 mg/kg/day to 60 mg/kg/day. In an embodiment of the present invention, hydroxyurea, lenalidomide, or palbociclib, as granulopoiesis inhibitors, was administered at a dose of 25 mg/kg/day, 30 mg/kg/day, 50 mg/kg/ day, 60 mg/kg/day, or 100 mg/kg/day. Depending on the dosage, the granulopoiesis inhibitor may be administered in divided doses several times a day. Specifically, the granulopoiesis inhibitor may be administered 1 to 4 times a day or 1 to 2 times a day.

The pharmaceutical composition may further include an immune checkpoint inhibitor (ICI).

The immune checkpoint inhibitor refers to a substance that inhibits the mechanism of cancer cells that interferes with the activation of T cells, and may be any one selected from the group consisting of an anti-PD-Ll antibody, an anti-PD-1 antibody, an anti-CTLA4 antibody, an anti PD-L2 antibody, an LTF2 modulating antibody, an anti-LAG3 antibody, an anti-A2aR antibody, an anti-TIGIT antibody, an anti-TIM-3 antibody, an anti-B7-H3 antibody, an anti-B7-H4 antibody, an anti-VISTA antibody, an anti-CD47 antibody, an anti-BTLA antibody, an anti-KIR antibody, an anti-IDO antibody, and a combination thereof.

Cancer cells hijack the immune checkpoint system as a mechanism to evade an immune response. Specifically, cancer cells use immune checkpoint receptors to evade immune responses, and representative receptors include PD-L1, PD-1, CTLA-4, *etc.* In order to prevent immune evasion of these cancer cells, immune checkpoint inhibitors, which are molecules that specifically bind to immune checkpoint receptors, are used for cancer treatment. The first immune checkpoint inhibitor is ipilimumab (Yervoy^{®}), which is a monoclonal antibody that specifically binds to cytotoxic T-lymphocytes associated antigen-4 (CTLA-4). The immune checkpoint therapy developed next is monoclonal antibodies against programmed cell death-1 (PD-1) and the corresponding ligand, programmed death ligand-1 (PD-L1). Representative drugs include anti-PD-1 antibodies (e.g., nivolumab (Opdivo^{®}), pembrolizumab (Keytruda^{®}), *etc.)* and anti-PD-Ll antibodies *(e.g.,* avelumab (Bavencio^{®}), atezolizumab (Tecentriq^{®}), durvalumab (Imfinzi^{®}), *etc.).*

In addition thereto, studies on monoclonal antibodies that specifically bind to various immune checkpoint receptors (*e*.*g*., glucocorticoid-induced TNFR-related protein (GITR), killer cell immunoglobulin-like receptor (KIR), lymphocyte-activation gene-3 (LAG-3), T-cell immunoglobulin and mucin-domain containing-3 (TIM-3), tumor-necrosis factor receptor superfamily member 4 (TNFRSF4)) are underway.

The dose of the immune checkpoint inhibitor may be administered in compliance with the dosage regimen of each manufacturer. The dose of the immune checkpoint inhibitor may be 0.1 mg/kg to 10 mg/kg or 1 mg/kg to 5 mg/kg. For example, in the case of Opdivo Inj. containing the nivolumab as an active ingredient, 3 mg/kg may be intravenously instilled over 60 minutes at intervals of 2 weeks, and regarding the dosage regimen as a combination therapy, 1 mg/kg may be intravenously instilled over 30 minutes. In addition, in the case of Keytruda Inj. containing pembrolizumab as an active ingredient, 200 mg may be intravenously instilled over 30 minutes at intervals of 3 weeks. As such, since even the same anti-PD-1 antibody has different dosage regimen depending on the product, it is preferred that the antibody be administerd in compliance with the manufacturer's dosage regimen.

When the pharmaceutical composition further includes an immune checkpoint inhibitor, the oncolytic virus, granulopoiesis inhibitor, and immune checkpoint inhibitor included in the pharmaceutical composition may be administered simultaneously, sequentially, or in reverse order.

Specifically, the oncolytic virus, granulopoiesis inhibitor, and immune checkpoint inhibitor may be administered simultaneously. In addition, the granulopoiesis inhibitor may be administered first, and the immune checkpoint inhibitor may be administered thereafter, and then the oncolytic virus may be administered. The granulopoiesis inhibitor may be administered first, and the oncolytic virus may be administered thereafter, and then the immune checkpoint inhibitor may be administered. The granulopoiesis inhibitor may be administered first, and then, the oncolytic virus and the immune checkpoint inhibitor may be administered simultaneously.

Furthermore, the oncolytic virus may be administered first, and the granulopoiesis inhibitor may be administered thereafter, and then the immune checkpoint inhibitor may be administered. The oncolytic virus may be administered first, and the immune checkpoint inhibitor may be administered thereafter, and then the granulopoiesis inhibitor may be administered. The oncolytic virus may be administered first, and then the granulopoiesis inhibitor and the immune checkpoint inhibitor may be administered simultaneously.

In addition, the immune checkpoint inhibitor may be administered first, and the granulopoiesis inhibitor may be administered thereafter, and then the oncolytic virus may be administered. The immune checkpoint inhibitor may be administered first, and the oncolytic virus may be administered thereafter, and then the granulopoiesis inhibitor may be administered. The immune checkpoint inhibitor may be administered first, and then the oncolytic virus and the granulopoiesis inhibitor may be administered simultaneously.

Furthermore, the granulopoiesis inhibitor may be administered first, and the oncolytic virus may be administered thereafter, and then the immune checkpoint inhibitor may be administered, and once again the granulopoiesis inhibitor may be administered. The granulopoiesis inhibitor may be administered first, and the immune checkpoint inhibitor may be administered thereafter, and then the oncolytic virus may be administered, and once again the granulopoiesis inhibitor may be administered. The granulopoiesis inhibitor may be administered first, and the oncolytic virus and the immune checkpoint inhibitor may be administered simultaneously, and once again the granulopoiesis inhibitor may be administered.

In addition, the granulopoiesis inhibitor may be administered first, and the oncolytic virus may be administered thereafter, and once again the granulopoiesis inhibitor may be administered, and then the immune checkpoint inhibitor may be administered. The granulopoiesis inhibitor may be administered first, and the immune checkpoint inhibitor may be administered thereafter, and once again the granulopoiesis inhibitor may be administered, and then the oncolytic virus may be administered.

Furthermore, the granulopoiesis inhibitor may be administered first, and the oncolytic virus may be administered thereafter, and once again the granulopoiesis inhibitor may be administered, and then the immune checkpoint inhibitor may be administered, and once again the granulopoiesis inhibitor may be administered. The granulopoiesis inhibitor may be administered first, and the immune checkpoint inhibitor may be administered thereafter, and once again the granulopoiesis inhibitor may be administered, and then the oncolytic virus may be administered, and once again the granulopoiesis inhibitor may be administered.

In addition, the oncolytic virus may be administered first, and the granulopoiesis inhibitor may be administered thereafter, and then the immune checkpoint inhibitor may be administered, and once again the granulopoiesis inhibitor may be administered. The immune checkpoint inhibitor may be administered first, and the granulopoiesis inhibitor may be administered thereafter, and then the oncolytic virus may be administered, and once again the granulopoiesis inhibitor may be administered.

The cancer may be solid cancer or blood cancer. Specifically, the blood cancer may be any one selected from the group consisting of lymphoma, acute leukemia, and multiple myeloma. The solid cancer may be any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, and combinations thereof.

In addition, the pharmaceutical composition of the present invention may further comprise a physiologically acceptable carrier. In addition, the pharmaceutical composition of the present invention may further comprise suitable excipients and diluents commonly used in the preparation of pharmaceutical compositions. In addition, the pharmaceutical composition may be formulated in the form of an injection according to a conventional method.

In a case of being formulated as preparations for parenteral administration, the pharmaceutical composition may be formulated into sterilized aqueous solutions, non-aqueous solutions, suspensions, emulsions, freeze-dried preparations, suppositories, or the like. For the non-aqueous solution or the suspension, propylene glycol, polyethylene glycol, vegetable oil such as olive oil, injectable ester such as ethyl oleate, or the like may be used. As the base of the suppository, Witepsol^{™}, macrogol, Tween^{™} 61, cacao butter, laurin fat, glycerogelatin, or the like may be used.

Regarding the administration route, dosage, and frequency of administration, the pharmaceutical composition may be administered to a subject in a variety of ways and amounts depending on the patient's condition and the presence or absence of side effects; and the optimal administration route, dosage, and frequency of administration therefor may be selected by a person skilled in the art within a suitable range. In addition, the pharmaceutical composition may be administered in combination with another drug or physiologically active substance whose therapeutic effect is known for the disease to be treated, or may be formulated in the form of a combination preparation with the other drug.

The pharmaceutical composition may be administered parenterally, and such administration may be performed by any suitable method, such as intratumoral, intraperitoneal, subcutaneous, intradermal, intranodal, intravenous, or intraarterial administration. Among these, intratumoral, intraperitoneal, or intravenous administration may be preferred. On the other hand, the dosage of the pharmaceutical composition may be determined depending on the administration schedule, the total dosage, and the patient's health condition.

The pharmaceutical composition for treating cancer may be characterized by increased cancer selectivity of the vaccinia virus.

Another aspect of the present invention provides a kit for preventing or treating cancer, which includes a first composition including a vaccinia virus as an active ingredient and a second composition including a granulopoiesis inhibitor as an active ingredient. The kit may further include a third composition which includes an immune checkpoint inhibitor as an active ingredient.

The vaccinia virus, granulopoiesis inhibitor, and immune checkpoint inhibitor are the same as those described above in the pharmaceutical composition.

The second composition that includes the granulopoiesis inhibitor as an active ingredient may be a commercialized drug. Examples of the commercialized drug that contains hydroxyurea as an active ingredient as the granulopoiesis inhibitor may include Hydroxyurea^{®}, Hydrea^{®}, Droxia^{™}, Mylocel^{™}, Siklos^{®}, and Hydrine^{®} capsule. The second composition may be taken orally, and parenteral administration thereof is also possible.

A dosage of the first composition varies depending on the individual's condition and body weight, the severity of disease, the type of drug, the route and period of administration, and may be appropriately selected by a person skilled in the art. The dosage may be such that a patient receives a vaccinia virus at 1×10⁵ to 1×10¹⁸ of virus particles, infectious virus units (TCID₅₀), or plaque forming units (pfu). Specifically, the dosage may be such that a patient receives a vaccinia virus at 1×10⁵, 2×10⁵, 5×10⁵, 1×10⁶, 2×10⁶, 5×10⁶, 1×10⁷, 2×10⁷, 5×10⁷, 1×10⁸, 2×10⁸, 5×10⁸, 1×10⁹, 2×10⁹, 5×10⁹, 1×10¹⁰, 5×10¹⁰, 1×10¹¹, 5×10¹¹, 1×10¹², 1×10¹³, 1×10¹⁴, 1×10¹⁵, 1×10¹⁶, 1×10¹⁷, or higher of virus particles, infectious virus units, or plaque forming units, and various numerical values and ranges between the above-mentioned numerical values may also be included therein. Preferably, the first composition may be administered at a dose of 1×10⁵ to 1×10¹⁰ pfu. More preferably, the first composition may be administered at a dose of equal to or greater than 1×10⁵ and lower than 1×10⁹ pfu. In an embodiment of the present invention, the first composition was administered at 1×10⁵ or 1×10⁷ pfu.

In addition, the second composition may be administered at a dose of 1 mg/kg/day to 100 mg/kg/day, or 10 mg/kg/day to 90 mg/kg/day. Specifically, the second composition may be administered at a dose of 10 mg/kg/day to 90 mg/kg/day, 15 mg/kg/day to 80 mg/kg/day, 20 mg/kg/day to 70 mg/kg/day, 25 mg/kg/day to 65 mg/kg/day, or 30 mg/kg/day to 60 mg/kg/day. In an embodiment of the present invention, the second composition was administered at 25 mg/kg/day, 30 mg/kg/day, 50 mg/kg/day, 60 mg/kg/day or 100 mg/kg/day. Depending on the dosage, the second composition may be administered in divided doses several times a day. Specifically, the second composition may be administered 1 to 4 times a day or 1 to 2 times a day.

The dose of the third composition may be administered in compliance with the dosage regimen of each manufacturer of the immune checkpoint inhibitor included in the third composition. The dose of the third composition may be 0.1 mg/kg to 10 mg/kg or 1 mg/kg to 5 mg/kg.

The cancer may be solid cancer or blood cancer. Specifically, the blood cancer may be any one selected from the group consisting of lymphoma, acute leukemia, and multiple myeloma. The solid cancer may be any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, and combinations thereof.

The first composition, the second composition and the third composition may further comprise a physiologically acceptable carrier. In addition, the composition included in the kit of the present invention may further comprise suitable excipients and diluents commonly used in the preparation of pharmaceutical compositions. In addition, the compositions may be formulated in the form of an injection according to a conventional method.

In a case of being formulated as preparations for parenteral administration, the first composition, the second composition and the third composition may be formulated into sterilized aqueous solutions, non-aqueous solutions, suspensions, emulsions, freeze-dried preparations, suppositories, or the like. For the non-aqueous solution or the suspension, propylene glycol, polyethylene glycol, vegetable oil such as olive oil, injectable ester such as ethyl oleate, or the like may be used. As the base of the suppository, Witepsol^{™}, macrogol, Tween^{™} 61, cacao butter, laurin fat, glycerogelatin, or the like may be used.

Regarding the administration route, dosage, and frequency of administration, the first composition, the second composition and the third composition may be administered to a subject in a variety of ways and amounts depending on the patient's condition and the presence or absence of side effects; and the optimal administration route, dosage, and frequency of administration therefor may be selected by a person skilled in the art within a suitable range. In addition, the pharmaceutical composition may be administered in combination with another drug or physiologically active substance whose therapeutic effect is known for the disease to be treated, or may be formulated in the form of a combination preparation with the other drug.

The second composition may be administered orally or parenterally. Specifically, the second composition may be administered parenterally, and such administration may be performed by intraperitoneal, intraarterial, or intravenous administration.

The first composition may be administered parenterally, and such administration may be performed by any suitable method, such as intratumoral, intraperitoneal, subcutaneous, intradermal, intranodal, intraarterial, or intravenous administration. Among these, intratumoral, intraperitoneal, or intravenous administration may be preferred. On the other hand, dosages of the first composition and the second composition may be determined depending on the administration schedule, the total dosage, and the patient's health condition.

In addition, the first composition may be administered 1 to 10 times or 2 to 5 times, and administration thereof to an individual may be performed at intervals of 7 to 30 days. Specifically, the first composition may be administered at intervals of 7 days, 14 days, 21 days, or 30 days.

The second composition may be administered before or after administration of the first composition. Specifically, the second composition may be continuously administered once a day starting from 3 to 5 days before administration of the first composition, and may be continuously administered once a day for 9 to 28 days starting from within 24 hours of or after 24 hours of administration of the first composition. In an embodiment of the present invention, the second composition may be continuously administered once a day starting from 1 to 3 days before administration of the first composition, and may be administered once a day for 13 days, 17 days, 18 days, or 28 days after administration of the first composition.

The third composition may be continuously administered for 1 to 10 weeks at least once a week after administering the first composition. Specifically, the third composition may be continuously administered for 1 to 8 weeks at least twice a week after administering the first composition.

In yet another aspect of the present invention, there is provided a method for treating cancer, comprising administering, to an individual having cancer, a vaccinia virus and granulopoiesis inhibitor.

The treatment method may further comprise administering an immune checkpoint inhibitor to an individual having cancer.

The oncolytic virus, granulopoiesis inhibitor, and immune checkpoint inhibitor are the same as described above in the pharmaceutical composition.

The vaccinia virus may belong to, but is not limited to, Western Reserve, NYVAC, Wyeth, LC16m8, Lister, Copenhagen, Tiantan, USSR, Tashkent, Evans, IHD-J, or IHD-W vaccinia virus strain.

The vaccinia virus and granulopoiesis inhibitor may be administered in combination simultaneously, sequentially, or in reverse order. Specifically, the vaccinia virus and granulopoiesis inhibitor may be administered simultaneously. In addition, the granulopoiesis inhibitor may be first administered, followed by the vaccinia virus. Furthermore, the vaccinia virus may be first administered, followed by the granulopoiesis inhibitor. In addition, the granulopoiesis inhibitor may be first administered, followed by the vaccinia virus, and then the granulopoiesis inhibitor may be administered again.

In addition, when the treatment method further includes administering an immune checkpoint inhibitor, the oncolytic virus, granulopoiesis inhibitor, and immune checkpoint inhibitor may be administered simultaneously, sequentially, or in reverse order. Specifically, the oncolytic virus, granulopoiesis inhibitor, and immune checkpoint inhibitor may be administered simultaneously. In addition, the granulopoiesis inhibitor may be administered first, and the immune checkpoint inhibitor may be administered thereafter, and then the oncolytic virus may be administered. The granulopoiesis inhibitor may be administered first, and the oncolytic virus may be administered thereafter, and then the immune checkpoint inhibitor may be administered. The granulopoiesis inhibitor may be administered first, and then the oncolytic virus and the immune checkpoint inhibitor may be administered simultaneously.

Furthermore, the oncolytic virus may be administered first, and the granulopoiesis inhibitor may be administered thereafter, and then the immune checkpoint inhibitor may be administered. The oncolytic virus may be administered first, and the immune checkpoint inhibitor may be administered thereafter, and then the granulopoiesis inhibitor may be administered. The oncolytic virus may be administered first, and then the granulopoiesis inhibitor and the immune checkpoint inhibitor may be administered thereafter, and then the granulopoiesis inhibitor may be administered simultaneously.

In addition, the immune checkpoint inhibitor may be administered first, and the granulopoiesis inhibitor may be administered thereafter, and then the oncolytic virus may be administered. The immune checkpoint inhibitor may be administered first, and the oncolytic virus may be administered thereafter, and then the granulopoiesis inhibitor may be administered. The immune checkpoint inhibitor may be administered first, and then the oncolytic virus and the granulopoiesis inhibitor may be administered simultaneously.

Furthermore, the granulopoiesis inhibitor may be administered first, and the oncolytic virus may be administered thereafter, and then the immune checkpoint inhibitor may be administered, and once again the granulopoiesis inhibitor may be administered. The granulopoiesis inhibitor may be administered first, and the immune checkpoint inhibitor may be administered thereafter, and then the oncolytic virus may be administered, and once again the granulopoiesis inhibitor may be administered. The granulopoiesis inhibitor may be administered first, and then the oncolytic virus and the immune checkpoint inhibitor may be administered simultaneously, and once again the granulopoiesis inhibitor may be administered.

In addition, the granulopoiesis inhibitor may be administered first, and the oncolytic virus may be administered thereafter, and once again the granulopoiesis inhibitor may be administered, and then the immune checkpoint inhibitor may be administered. The granulopoiesis inhibitor may be administered first, and the immune checkpoint inhibitor may be administered thereafter, and once again the granulopoiesis inhibitor may be administered, and then the oncolytic virus may be administered.

Furthermore, the granulopoiesis inhibitor may be administered first, and the oncolytic virus may be administered thereafter, and once again the granulopoiesis inhibitor may be administered, and then the immune checkpoint inhibitor may be administered, and once again the granulopoiesis inhibitor may be administered. The granulopoiesis inhibitor may be administered first, and the immune checkpoint inhibitor may be administered thereafter, and once again the granulopoiesis inhibitor may be administered, and then the oncolytic virus may be administered, and once again the granulopoiesis inhibitor may be administered.

In addition, the oncolytic virus may be administered first, and the granulopoiesis inhibitor may be administered thereafter, and then the immune checkpoint inhibitor may be administered, and once again the granulopoiesis inhibitor may be administered. The immune checkpoint inhibitor may be administered first, and the granulopoiesis inhibitor may be administered thereafter, and then the oncolytic virus may be administered, and once again the granulopoiesis inhibitor may be administered.

A dosage of the vaccinia virus varies depending on the individual's condition and body weight, the severity of disease, the type of drug, the route and period of administration, and may be appropriately selected by a person skilled in the art. The dosage may be such that a patient receives a vaccinia virus at 1×10⁵ to 1×10¹⁸ of virus particles, infectious virus units (TCID₅₀), or plaque forming units (pfu). Specifically, the dosage may be such that a patient receives a vaccinia virus at 1×10⁵, 2×10⁵, 5×10⁵, 1×10⁶, 2×10⁶, 5×10⁶, 1×10⁷, 2×10⁷, 5×10⁷, 1×10⁸, 2×10⁸, 5×10⁸, 1×10⁹, 2×10⁹, 5×10⁹, 1×10¹⁰, 5×10¹⁰, 1×10¹¹, 5×10¹¹, 1×10¹², 1×10¹³, 1×10¹⁴, 1×10¹⁵, 1×10¹⁶, 1×10¹⁷, or higher of virus particles, infectious virus units, or plaque forming units, and various numerical values and ranges between the above-mentioned numerical values may also be included therein. Preferably, the vaccinia virus may be administered at a dose of 1×10⁵ to 1×10¹⁰ pfu. More preferably, the vaccinia virus may be administered at a dose of equal to or greater than 1×10⁵ and lower than 1×10⁹ pfu. In an embodiment of the present invention, the vaccinia virus was administered at 1×10⁵ or 1×10⁷ pfu.

In addition, the granulopoiesis inhibitor may be administered at a dose of 1 mg/kg/day to 100 mg/kg/day, or 10 mg/kg/day to 90 mg/kg/day. Specifically, the granulopoiesis inhibitor may be administered at a dose of 10 mg/kg/day to 90 mg/kg/day, 15 mg/kg/day to 80 mg/kg/day, 20 mg/kg/day to 70 mg/kg/day, 25 mg/kg/day to 65 mg/kg/day, or 30 mg/kg/day to 60 mg/kg/day. In an embodiment of the present invention, hydroxyurea, lenalidomide, or palbociclib, as granulopoiesis inhibitors, was administered at a dose of 25 mg/kg/day, 30 mg/kg/day, 50 mg/kg/ day, 60 mg/kg/day, or 100 mg/kg/day. Depending on the dosage, the granulopoiesis inhibitor may be administered in divided doses several times a day. Specifically, the granulopoiesis inhibitor may be administered 1 to 4 times a day or 1 to 2 times a day.

In addition, the vaccinia virus may be administered 1 to 10 times or 2 to 5 times, and may be administered to an individual at intervals of 7 to 30 days. Specifically, the vaccinia virus may be administered at intervals of 7 days, 14 days, 21 days, or 30 days.

The granulopoiesis inhibitor may be administered before, during, or after administration of the vaccinia virus. Specifically, the granulopoiesis inhibitor may be administered before or after administration of the vaccinia virus. The granulopoiesis inhibitor may be continuously administered once a day starting from 3 to 5 days before administration of the vaccinia virus, and may be continuously administered once a day for 9 to 28 days starting from 24 hours after administration of the vaccinia virus. In an embodiment of the present invention, the granulopoiesis inhibitor may be continuously administered once a day starting from 1 to 3 days before administration of the vaccinia virus, and may be administered once a day for 13 days, 17 days, 18 days, or 28 days after administration of the vaccinia virus.

The cancer may be solid cancer or blood cancer. Specifically, the blood cancer may be any one selected from the group consisting of lymphoma, acute leukemia, and multiple myeloma. The solid cancer may be any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, and combinations thereof.

The granulopoiesis inhibitor may be administered orally or parenterally. Specifically, the granulopoiesis inhibitor may be administered parenterally, and such administration may be performed by intraperitoneal, intraarterial, or intravenous administration.

The vaccinia virus and granulopoiesis inhibitor may be administered parenterally, and such administration may be performed by any suitable method, such as intratumoral, intraperitoneal, subcutaneous, intradermal, intranodal, intravenous, or intraarterial administration. Among these, intratumoral, intraperitoneal, or intravenous administration may be preferred. On the other hand, the dosages of the vaccinia virus and granulopoiesis inhibitor may be determined depending on the administration schedule, the total dosage, and the patient's health condition.

As used herein, the term "individual" refers to a person who has or is suffering from a disease in a state that may be alleviated, inhibited, or treated by administering the pharmaceutical composition of the present invention.

As used herein, the term "administration" means introducing an effective amount of a substance into an individual by an appropriate method, and administration of the vaccinia virus and the granulopoiesis inhibitor may be performed via a common route that allows the substances to reach a target tissue.

In addition, the vaccinia virus and the granulopoiesis inhibitor may be administered in combination with another drug or physiologically active substance whose therapeutic effect is known for the disease to be treated, or may be formulated in the form of a combination preparation with the other drug.

In still yet another aspect of the present invention, there is provided a use of a composition, which includes a vaccinia virus and granulopoiesis inhibitor, for the prevention or treatment of cancer.

In still yet another aspect of the present invention, there is provided a use of a composition, which includes a vaccinia virus and granulopoiesis inhibitor, for the manufacture of a medicament for preventing or treating cancer.

In still yet another aspect of the present invention, there is provided an anticancer adjuvant, comprising granulopoiesis inhibitor as an active ingredient. Here, the granulopoiesis inhibitor is as described above for the pharmaceutical composition. In addition, the anticancer adjuvant may be characterized in that it is used as an anticancer adjuvant for an anticancer agent that includes a vaccinia virus as an active ingredient. The anticancer adjuvant may be characterized in that it improves, enhances, or increases anticancer activity of the vaccinia virus. The anticancer adjuvant may be characterized in that it increases cancer selectivity of the vaccinia virus.

The granulopoiesis inhibitor may be hydroxyurea, lenalidomide, thalidomide, tadalafil, palbociclib, alkylating agents, anthracyclines, antimetabolites, camptothecins, epipodophyllotoxins, mitomycin C, taxanes, or vinblastine.

### Mode for the Invention

Hereinafter, the present invention will be described in more detail by way of examples. However, the following examples are for illustrative purposes only, and the scope of the present invention is not limited thereto.

### Preparation Example 1. Production of recombinant vaccinia viruses (Wyeth VV^{tk-}, WR VV^{tk-})

### Preparation Example 1.1. Construction of shuttle plasmid vector

To produce recombinant vaccinia viruses in which thymidine kinase (TK) gene is deleted, the wild-type vaccinia viruses, that is, Wyeth strain (NYC Department of Health) and Western Reserve strain were purchased from the American Type Culture Collection (ATCC). For recombination, a TK region in the wild-type vaccinia virus was subjected to substitution using a shuttle plasmid vector that contains firefly luciferase reporter (p7.5 promoter) gene or GFP gene.

### Preparation Example 1.2. Production of recombinant vaccinia viruses

To obtain recombinant viruses, HeLa cells (ATCC) were seeded in 6-well plates at 4×10⁵ cells per well, and then culture was performed in EMEM medium containing 10% fetal bovine serum. Subsequently, treatment with the wild-type vaccinia virus was performed at an MOI of 0.05. 2 hours later, the medium was replaced with EMEM medium containing 2% fetal bovine serum, and then the cells were transfected with 4 µg of the shuttle plasmid vector, which was constructed in Preparation Example 1.1 and linearized, using Xfect^{™} polymer (Clonetech 631317, USA). Culture was performed for 4 hours. Subsequently, the medium was replaced with EMEM medium containing 2% fetal bovine serum, and then culture was additionally performed for 72 hours. Finally, the infected cells were collected, and then freezing and thawing were repeated 3 times. Subsequently, the cells were lysed by sonication, and a sucrose cushion method was used to obtain free recombinant vaccinia viruses, which were designated Wyeth VV^{tk-} or WR VV^{tk-}.

### Preparation Example 2. Production of recombinant vaccinia virus (OTS-412)

To produce a recombinant vaccinia virus in which thymidine kinase (TK) gene is deleted and which expresses a mutated herpes simplex virus thymidine kinase (HSV-TK) gene, a TK region in the Wyeth strain wild-type vaccinia virus was subjected to substitution using as a shuttle vector pUC57amp+ plasmid (Genewiz, USA) into which synthesized mutated type 1 HSV-TK gene (pSE/L promoter) of SEQ ID NO: 1 and firefly luciferase reporter (p7.5 promoter) gene were recombined. A recombinant vaccinia virus was obtained in the same manner as in Preparation Example 1.2 using the shuttle vector as constructed above, and this virus was designated OTS-412.

### Preparation Example 3. Production of recombinant vaccinia virus (WOTS-418)

To produce a recombinant vaccinia virus in which thymidine kinase (TK) gene is deleted and which expresses a mutated herpes simplex virus thymidine kinase (HSV-TK) gene, a TK region in the Western Reserve strain wild-type vaccinia virus was subjected to substitution using as a shuttle vector pUC57amp+ plasmid (Genewiz, USA) into which synthesized mutated type 1 HSV-TK gene (pSE/L promoter) of SEQ ID NO: 2 and firefly luciferase reporter (p7.5 promoter) gene were recombined. A recombinant vaccinia virus was obtained in the same manner as in Preparation Example 1.2 using the shuttle vector as constructed above, and this virus was designated WOTS-418.

### Preparation Example 4. Production of recombinant vaccinia virus (VV_DD)

To produce a recombinant vaccinia virus in which thymidine kinase (TK) gene and vaccinia growth factor (VGF) gene are deleted, a TK region in the Western Reserve strain wild-type vaccinia virus was subjected to substitution using a shuttle plasmid that contains enhanced green fluorescent protein (EGFP) gene, and a VGF gene region in the same virus was subjected to substitution using a shuttle plasmid that contains lacZ gene. A recombinant vaccinia virus was obtained in the same manner as in Preparation Example 1.2 using the shuttle plasmid that contains EGFP gene and the shuttle plasmid that contains lacZ gene, and this virus was designated VV_DD.

### I. Identification of synergistic anticancer effect by co-administration of vaccinia virus and hydroxyurea

### Experimental Example 1. Identification of cancer therapeutic effect of wild-type vaccinia virus (WR) and hydroxyurea in mouse renal cancer cell-transplanted mice: Renca (I)

### Experimental Example 1.1. Production of mouse renal cancer cell-transplanted mice and drug administration

Balb/c mice (female, 10-week-old) purchased from ORIENT BIO (Busan, Korea) were subjected to a 2-day acclimatization period, and then subcutaneously transplanted with Renca cancer cell line (Korea Cell Line Bank) at 5×10⁶ cells. The tumor volume was observed until it reached 50 mm³ to 80 mm³, and then administration of a wild-type vaccinia virus was started. On the other hand, the Western Reserve strain wild-type vaccinia virus (WR) has stronger proliferative capacity in an allograft model than a Wyeth strain wild-type vaccinia virus.

The produced mouse renal cancer cell-transplanted mice were divided into 3 groups (n=6). The group receiving intraperitoneal administration of saline was set as a negative control group, and the group receiving administration of the wild-type vaccinia virus (WR, 1×10⁵ pfu) as a positive control group. In addition, the group receiving co-administration of the wild-type vaccinia virus (WR, 1×10⁵ pfu) and hydroxyurea (30 mg/kg) was set as an experimental group. The wild-type vaccinia virus was intratumorally administered once; and the hydroxyurea was intraperitoneally administered 5 times per week starting from 1 day before administration of the wild-type vaccinia virus to day 14 after the administration, except for the day of administration of the wild-type vaccinia virus.

### Experimental Example 1.2. Checking for changes in tumor volume

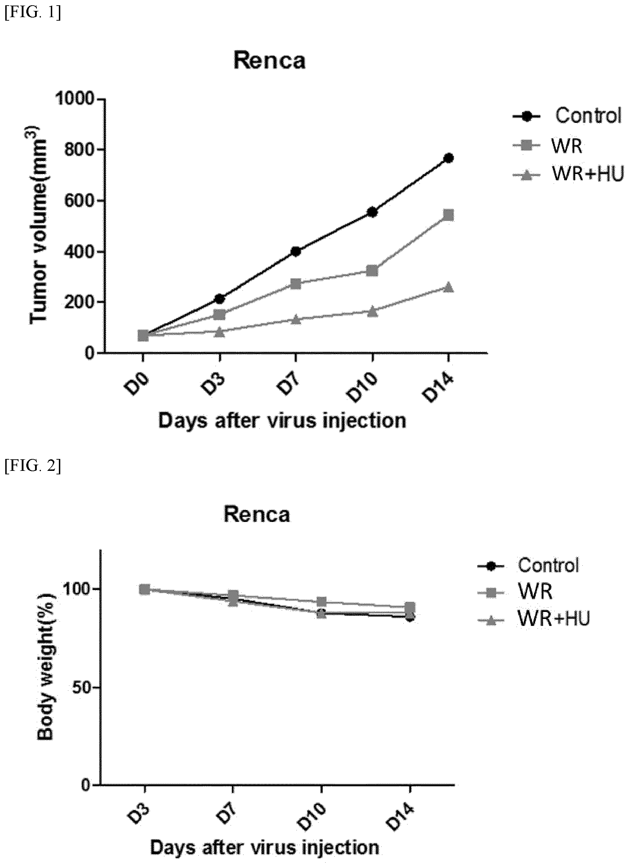

Tumor volumes were measured on days 0, 3, 7, 10, and 14 after the drug administration to the mice of each group in Experimental Example 1.1. As a result, it was identified that the tumor volume in the mice of the positive control group was suppressed as compared with the negative control group, whereas the tumor volume in the mice of the experimental group was remarkably suppressed (FIG. 1).

### Experimental Example 1.3. Checking for changes in body weight

Body weights were measured on days 3, 7, 10, and 14 after each drug administration to the mice of the negative control group, the positive control group, and the experimental group in Experimental Example 1.1. As a result, there was no significant body weight loss in all three groups (FIG. 2).

### Experimental Example 2. Identification of cancer therapeutic effect of recombinant vaccinia virus (WR VV^{tk-}) and hydroxyurea in mouse renal cancer cell-transplanted mice: Renca (II)

### Experimental Example 2.1. Production of mouse renal cancer cell-transplanted mice and drug administration

Balb/c mice (female, 8-week-old) purchased from ORIENT BIO (Busan, Korea) were subjected to a one-week acclimatization period, and then allografted with Renca cancer cell line (Korea Cell Line Bank) at 5×10⁶ cells. The tumor volume was observed until it reached 100 mm³ to 150 mm³, and then administration of a recombinant vaccinia virus was started. On the other hand, Western Reserve strain-derived recombinant vaccinia virus (WR VV^{tk-}) has stronger proliferative capacity in an allograft model than a Wyeth strain-derived recombinant vaccinia virus.

The produced mouse renal cancer cell-transplanted mice were divided into 3 groups (n=8). The group receiving intraperitoneal administration of saline was set as a negative control group, and the group receiving administration of recombinant vaccinia virus (WR VV^{tk-}, 1×10⁷ pfu) was set as a positive control group. In addition, the group receiving co-administration of the recombinant vaccinia virus and hydroxyurea (60 mg/kg) was set as an experimental group. The recombinant vaccinia virus was intratumorally administered twice; and the hydroxyurea was intraperitoneally administered 6 times per week starting from 1 day before administration of the recombinant vaccinia virus to day 21 after the administration, except for the day of administration of the recombinant vaccinia virus.

### Experimental Example 2.2. Checking for changes in tumor volume

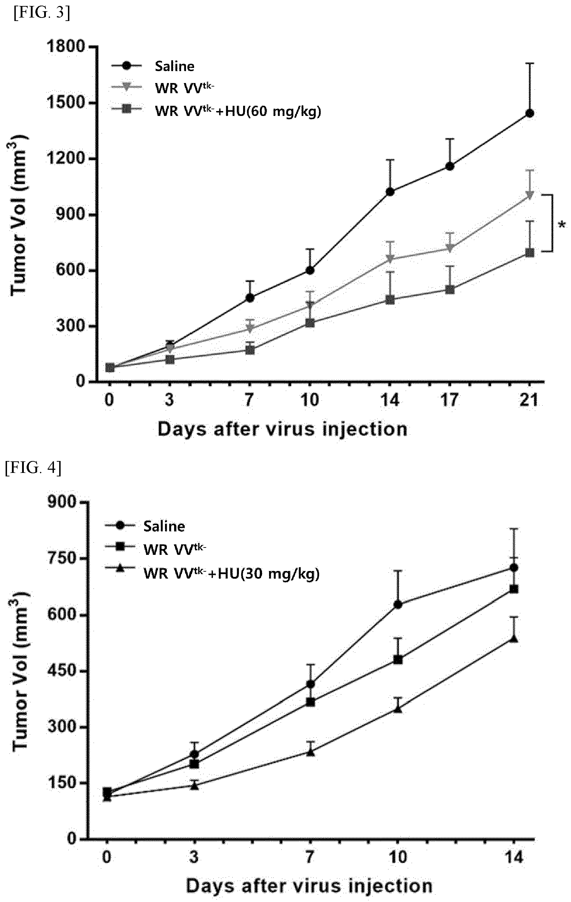

Tumor volumes were measured on days 0, 3, 7, 10, 14, 17, and 21 after the drug administration to the mice of each group in Experimental Example 2.1. As a result, it was identified that the tumor volume in the mice of the experimental group was significantly suppressed as compared with the tumor volume in the mice of the positive control group (FIG. 3).

### Experimental Example 3. Identification of cancer therapeutic effect of recombinant vaccinia virus (WR VV^{tk-}) and hydroxyurea in mouse renal cancer cell-transplanted mice: Renca (III)

Balb/c mice (female, 10-week-old) purchased from ORIENT BIO (Busan, Korea) were subjected to a 2-day acclimatization period, and then subcutaneously transplanted in the left thigh with Renca cancer cell line (Korea Cell Line Bank) at 5×10⁶ cells. The tumor volume was observed until it reached 50 mm³ to 150 mm³, and then administration of a recombinant vaccinia virus was started.

The produced mouse renal cancer cell-transplanted mice were divided into 3 groups (n=6). The group receiving intraperitoneal administration of saline was set as a negative control group, and the group receiving administration of a recombinant vaccinia virus (WR VV^{tk-}, 1×10⁵ pfu) as a positive control group. In addition, the group receiving co-administration of the recombinant vaccinia virus and hydroxyurea (30 mg/kg) was set as an experimental group. The recombinant vaccinia virus was intratumorally administered once; and the hydroxyurea was intraperitoneally administered 6 times per week starting from 1 day before administration of the recombinant vaccinia virus to day 14 after the administration, except for the day of administration of the recombinant vaccinia virus.

Tumor volumes were measured on days 0, 3, 7, 10, and 14 after the drug administration to the mice of each group. As a result, it was identified that the tumor volume in the mice of the experimental group was suppressed by about 25% in growth as compared with the tumor volume in the mice of the positive control group (FIG. 4).

### Experimental Example 4. Identification of cancer therapeutic effect of recombinant vaccinia virus (VV_DD) and hydroxyurea in mouse melanoma-transplanted mice: B16F10

C57BL/6 mice (female, 7-week-old) purchased from KOATECH (Korea) were subjected to a 2-day acclimatization period, and then subcutaneously transplanted with a mouse melanoma cancer cell line (ATCC, B16F10) at 5×10⁵ cells. The tumor volume was observed until it reached 50 mm³ to 100 mm³, and then administration of a recombinant vaccinia virus (VV DD) was started. The recombinant vaccinia virus (VV DD) was obtained by performing double deletion of thymidine kinase (TK) and vaccinia growth factor (VGF) regions in a Western Reserve strain vaccinia virus, and has limited proliferation capacity in an allograft model.

The produced mouse melanoma-transplanted mice were divided into 4 groups (n=6). The group receiving intraperitoneal administration of saline was set as a negative control group, and the group receiving administration of hydroxyurea (30 mg/kg) or the recombinant vaccinia virus (VV DD, 1×10⁶ pfu) alone as a positive control group. In addition, the group receiving co-administration of the recombinant vaccinia virus and hydroxyurea (30 mg/kg) was set as an experimental group. The recombinant vaccinia virus was intraperitoneally administered on days 0 and 5; and the hydroxyurea was intraperitoneally administered 6 times per week starting from 1 day before administration of the recombinant vaccinia virus to day 15 after the administration, except for the day of administration of the recombinant vaccinia virus.

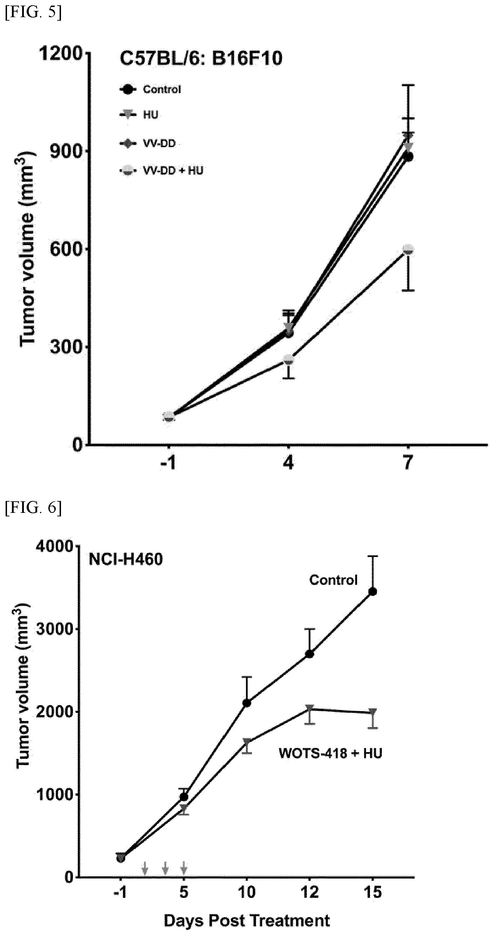

Tumor volumes were measured 1 day before drug administration to the mice of each group and days 4 and 7 after the administration. As a result, it was identified that the tumor volume in the mice of the experimental group was significantly suppressed as compared with the tumor volume in the mice of the positive control group (FIG. 5). From these results, it was identified that a synergistic effect was observed in a case where the recombinant vaccinia virus (VV DD) and the hydroxyurea were co-administered.

### Experimental Example 5. Identification of cancer therapeutic effect of recombinant vaccinia virus (WOTS-418) and hydroxyurea in human lung cancer cell line-transplanted mice: NCI-H460

Balb/c nu/nu mice (female, 7-week-old) purchased from ORIENT BIO (Busan, Korea) were subjected to a 2-day acclimatization period, and then subcutaneously xenografted with NCI-H460 human lung cancer cell line (Korea Cell Line Bank) at 5×10⁶ cells. The tumor volume was observed until it reached 100 mm³ to 150 mm³, and then administration of a recombinant vaccinia virus (WOTS-418) was started. On the other hand, the Western Reserve strain-derived recombinant vaccinia virus (WOTS-418) has proliferation capacity in human lung cancer cell line (NCI-H460)-xenografted mice.

The produced human lung cancer cell line-transplanted mice were divided into 2 groups (n=4). The group receiving intraperitoneal administration of saline was set as a control group, and the group receiving co-administration of the recombinant vaccinia virus (WOTS-418, 1×10⁷ pfu) and hydroxyurea (30 mg/kg) was set as an experimental group. The recombinant vaccinia virus was intraperitoneally administered once; and the hydroxyurea was intraperitoneally administered 6 times per week starting from 1 day before administration of the recombinant vaccinia virus to day 15 after the administration, except for the day of administration of the recombinant vaccinia virus.

Tumor volumes were measured on days 0, 5, 10, 12, and 15 after drug administration to the mice of each group. As a result, it was identified that the tumor volume in the mice of the experimental group was suppressed by about 40% as compared with the control group (FIG. 6).

### Experimental Example 6. Analysis of survival for recombinant vaccinia virus (WOTS-418) and hydroxyurea in mouse colorectal cancer cell-transplanted mice: CT-26

Balb/c mice (female, 7-week-old) purchased from ORIENT BIO (Busan, Korea) were subjected to a 2-day acclimatization period, and then subcutaneously transplanted with a mouse colorectal cancer cell line (CT-26, Korea Cell Line Bank) at 1×10⁶ cells. After 7 days, administration of a recombinant vaccinia virus (WOTS-418) and hydroxyurea was started. On the other hand, the Western Reserve strain-derived recombinant vaccinia virus (WOTS-418) has stronger proliferation capacity in an allograft model as compared with a Wyeth strain-derived recombinant vaccinia virus.

The produced mouse colorectal cancer cell line-transplanted mice were divided into 2 groups (n=12), that is, the group receiving intraperitoneal administration of the recombinant vaccinia virus (WOTS-418, 1×10⁷ pfu) and the group receiving co-administration of the recombinant vaccinia virus and hydroxyurea (30 mg/kg). The recombinant vaccinia virus was intraperitoneally administered once; and the hydroxyurea was intraperitoneally administered 5 times consecutively starting from day 1 after administration of the recombinant vaccinia virus.

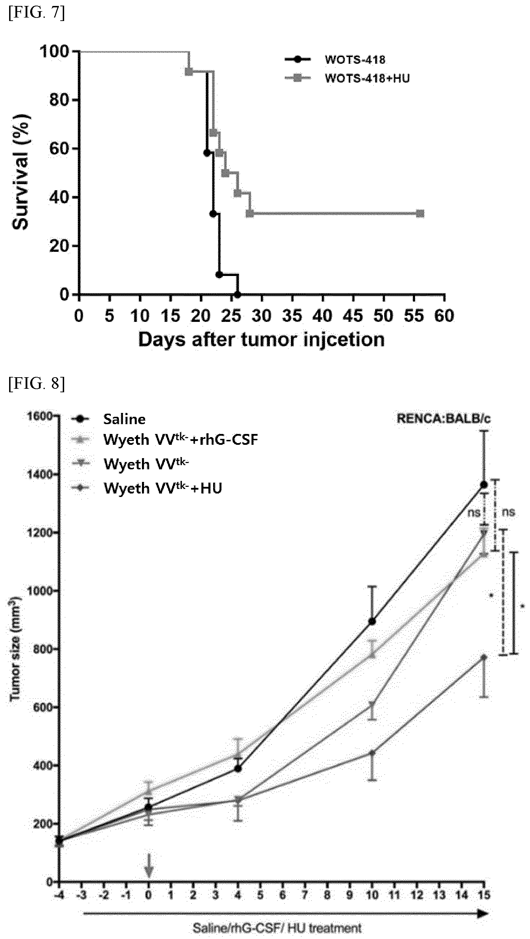

Survival curves for the mice of respective groups were analyzed. As a result, for the group having received administration of the recombinant vaccinia virus alone, all mice died 25 days after the administration; however, 30% or higher of the mice, which had received co-administration of the hydroxyurea and the recombinant vaccinia virus, survived for 55 days or longer (FIG. 7). From these results, it was identified that in a case where the recombinant vaccinia virus and hydroxyurea were co-administered, enhanced safety was obtained as compared with a case where only the recombinant vaccinia virus was administered.

### Experimental Example 7. Identification of cancer therapeutic effect of recombinant vaccinia virus (Wyeth VV^{tk-}) and hydroxyurea in mouse renal cancer cell-transplanted mice: Renca (IV)

### Experimental Example 7.1. Production of mouse renal cancer cell-transplanted mice and drug administration

Balb/c mice (female, 7-week-old) purchased from ORIENT BIO (Busan, Korea) were subjected to a 2-day acclimatization period, and then allografted with Renca cancer cell line (Korea Cell Line Bank) at 5×10⁶ cells. The tumor volume was observed until it reached 100 mm³ to 150 mm³, and then administration of a recombinant vaccinia virus was started.

The produced mouse renal cancer cell-transplanted mice were divided into 4 groups (n=4). The group receiving intratumoral administration of saline was set as a negative control group, and the group receiving administration of the recombinant vaccinia virus (Wyeth VV^{tk-}, 1×10⁷ pfu) as a positive control group. In addition, the group receiving co-administration of the recombinant vaccinia virus (Wyeth VV^{tk-}, 1×10⁷ pfu) and a recombinant human granulocyte colony-stimulating factor (rhG-CSF, 75 µg/kg) and the group receiving administration of the recombinant virus (Wyeth VV^{tk-}, 1×10⁷ pfu) and hydroxyurea (30 mg/kg) was set as experimental groups. The recombinant vaccinia virus was intratumorally administered, and rhG-CSF or the hydroxyurea was intraperitoneally administered 5 times per week starting from 3 days before administration of the recombinant vaccinia virus until sacrifice.

### Experimental Example 7.2. Checking for changes in tumor volume

The mice of each group in Experimental Example 7.1 were sacrificed on day 16 after drug administration, and tumor volumes were measured. As a result, the mice of the positive control group and the mice of the experimental group having received co-administration of the recombinant vaccinia virus and rhG-CSF showed a nearly 10-fold increase as compared with the initial tumor volume. On the other hand, the mice of the experimental group having received co-administration of the recombinant vaccinia virus and hydroxyurea showed a nearly 8-fold increase as compared with the initial tumor volume, and this was the most suppressed tumor volume observed (FIG. 8).

### Experimental Example 7.3. Identification of antigen-specific cytotoxic T lymphocyte (CTL) activation

To identify whether a tumor-specific anticancer effect is obtained in a case where a recombinant vaccinia virus and hydroxyurea are co-administered, the mice of each group in Experimental Example 7.1 were sacrificed on day 16, and then lymphocytes in the spleen were isolated from each group. Then, the isolated lymphocytes were injected respectively into new normal mice. Cancer transplantation was performed and tumor volumes were observed. Specifically, one week later, the mice were allografted with Renca cancer cell line (Korea Cell Line Bank) at 5×10⁶ cells, and tumor volumes were measured on day 19.

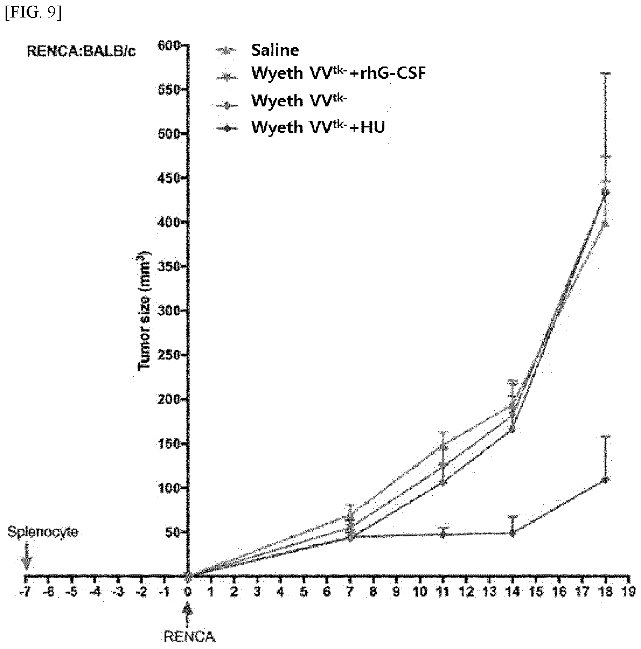

As a result, tumor growth was remarkably suppressed in the mice injected with the splenocytes collected from the mice of the group having received co-administration of the recombinant vaccinia virus and hydroxyurea. On the other hand, tumor growth was not significantly suppressed in each of the mice injected with the splenocytes collected from the mice of the remaining groups (FIG. 9). From these results, it was identified that for the group having received co-administration of the recombinant vaccinia virus and hydroxyurea, not only immune cells such as cytotoxic T cells were produced, but also adaptive immunity was activated.

### Experimental Example 8. Identification of cancer therapeutic effect of recombinant vaccinia virus (Wyeth VV^{tk-}) and hydroxyurea in mouse renal cancer cell-transplanted mice: Renca (V)

### Experimental Example 8.1. Production of mouse renal cancer cell-transplanted mice and drug administration

Balb/c mice (female, 7-week-old) purchased from ORIENT BIO (Busan, Korea) were subjected to a one-week acclimatization period, and then allografted with Renca cancer cell line (Korea Cell Line Bank) at 5×10⁶ cells. The tumor volume was observed until it reached 50 mm³ to 100 mm³, and then administration of a recombinant vaccinia virus was started. On the other hand, the Wyeth strain-derived recombinant vaccinia virus (Wyeth VV^{tk-}) hardly proliferates in a mouse renal cancer cell-transplanted mouse model.

The produced mouse renal cancer cell-transplanted mice were divided into 4 groups (n=4). The group receiving intratumoral administration of saline was set as a negative control group, and the group receiving administration of hydroxyurea (30 mg/kg) alone and the group receiving administration of the recombinant vaccinia virus (Wyeth VV^{tk-}, 1×10⁷ pfu) alone were set as positive control groups. In addition, the group receiving co-administration of the recombinant vaccinia virus (Wyeth VV^{tk-}, 1×10⁷ pfu) and hydroxyurea (30 mg/kg) was set as an experimental group. The recombinant vaccinia virus was intratumorally administered, and the hydroxyurea was intraperitoneally administered 5 times per week starting from 3 days before administration of the recombinant vaccinia virus until sacrifice.

### Experimental Example 8.2. Checking for changes in tumor volume

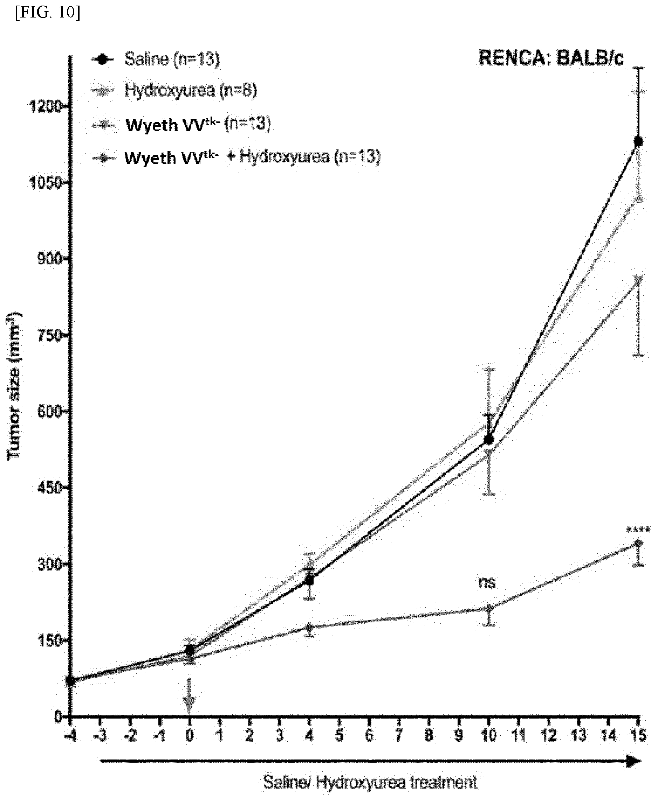

Tumor volumes were measured on days 0, 4, 10, 15, and 22 after the drug administration to the mice of each group in Experimental Example 8.1. As a result, the tumor volume in the mice of the positive control group increased by about 11 to 13 fold as compared with the initial tumor volume. On the other hand, the tumor volume in the mice of the experimental group increased by about 4 fold as compared with the initial tumor volume (FIG. 10).

### Experimental Example 8.3. Identification of tumor-specific cytotoxic T lymphocyte (CTL) activation

To identify whether a tumor-specific anticancer effect is obtained in a case where a recombinant vaccinia virus and hydroxyurea are co-administered, the mice of each group in Experimental Example 8.1 were sacrificed on day 16, and then splenocytes and cytotoxic T lymphocytes (CD8+ T cells) were isolated from each group. Then, the isolated splenocytes or cytotoxic T lymphocytes were injected respectively into new normal mice. Cancer transplantation was performed and tumor volumes were observed. Specifically, one week later, the mice were allografted with Renca cancer cell line (Korea Cell Line Bank) at 5×10⁶ cells, and tumor volumes were measured on days 7, 10, 14, 18, and 21.

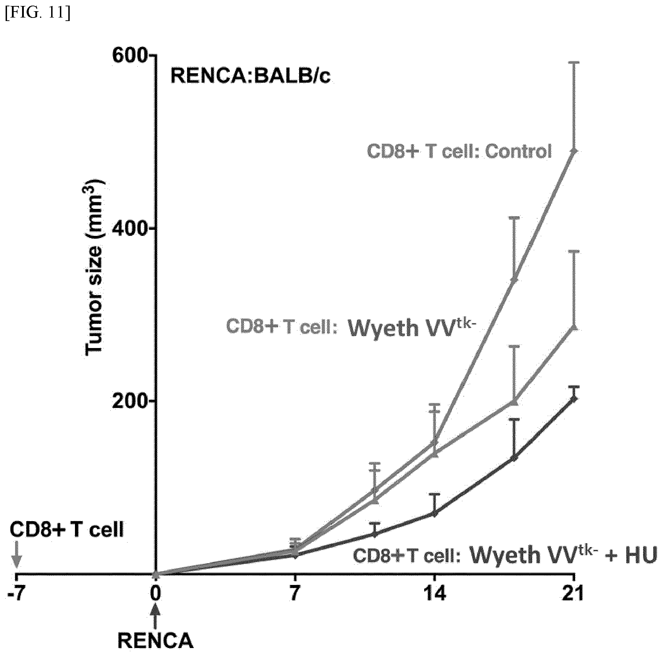

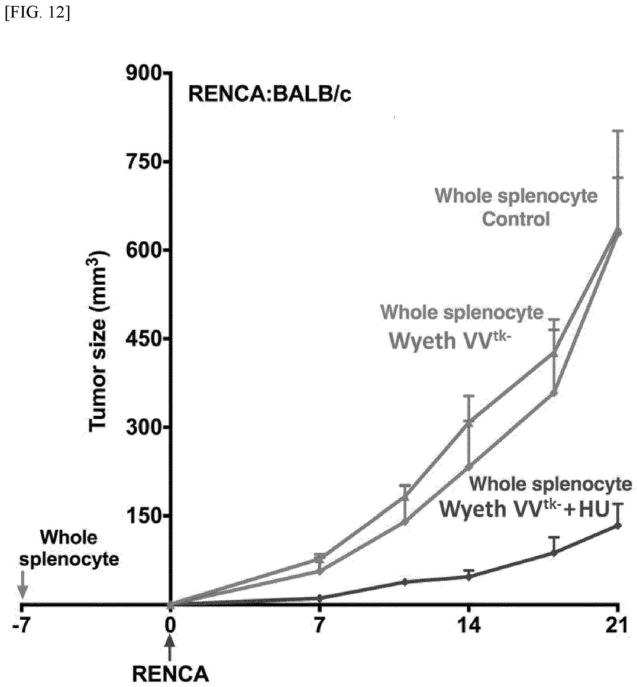

As a result, tumor growth was remarkably suppressed in the mice injected with the splenocytes or T lymphocytes collected from the mice of the experimental group. On the other hand, tumor growth was not significantly suppressed in the mice injected with the splenocytes or T lymphocytes collected from the mice of the remaining groups (FIG. 11). From these results, it was identified that for the group having received co-administration of the recombinant vaccinia virus and hydroxyurea, adaptive immunity with anticancer efficacy was activated not only due to T lymphocytes but also other immune cells formed in the spleen (FIGS. 11 and 12).

### Experimental Example 9. Identification of cancer therapeutic effect of recombinant vaccinia virus (Wyeth VV^{tk-}) and hydroxyurea in mouse renal cancer cell-transplanted mice: Renca (VI)

### Experimental Example 9.1. Production of mouse renal cancer cell-transplanted mice and drug administration

Balb/c mice (female, 8-week-old) purchased from ORIENT BIO (Busan, Korea) were subjected to a one-week acclimatization period, and then allografted with Renca cancer cell line (Korea Cell Line Bank) at 5×10⁶ cells. The tumor volume was observed until it reached 100 mm³ to 150 mm³, and then administration of a recombinant vaccinia virus was started. On the other hand, the Wyeth strain-derived recombinant vaccinia virus (Wyeth VV^{tk-}) hardly proliferates in a mouse renal cancer cell-transplanted mouse model.

The produced mouse renal cancer cell-transplanted mice were divided into 3 groups (n=6). The group receiving intratumoral administration of saline was set as a negative control group, and the group receiving administration of the recombinant vaccinia virus (Wyeth VV^{tk-}, 1×10⁷ pfu) as a positive control group. In addition, the group receiving administration of the recombinant vaccinia virus (Wyeth VV^{tk-}, 1×10⁷ pfu) and hydroxyurea (30 mg/kg) was set as an experimental group. The recombinant vaccinia virus was intratumorally administered, and the hydroxyurea was intraperitoneally administered 6 times per week starting from 1 day before administration of the recombinant vaccinia virus until sacrifice.

### Experimental Example 9.2. Checking for changes in tumor volume

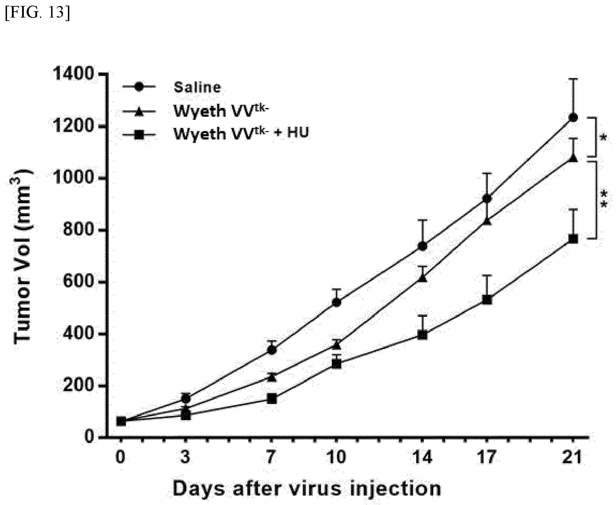

The mice of each group in Experimental Example 9.1 were sacrificed on day 22 after drug administration, and tumor volumes were measured. As a result, the tumor volume in the mice of the positive control group was suppressed by about 25% as compared with the tumor volume in the mice of the negative control group. In particular, the tumor volume in the mice of the experimental group was suppressed by about 37.5% as compared with the tumor volume in the mice of the negative control group, and was suppressed by about 15% as compared with the tumor volume in the mice of the positive control group (FIG. 13).

### Experimental Example 9.3. Identification of spleen tissue microenvironment

Distribution of immune cells in the tumor microenvironment was analyzed when a recombinant vaccinia virus and hydroxyurea were co-administered. For analysis, immunohistochemical staining using diaminobenzidine (DAB) was performed. Specifically, the spleen was collected from the mice of each group. The spleen tissue was cut into 0.4 µm and dried. Subsequently, the tissue was washed with PBS, and then treated with bovine serum albumin (BSA). The tissue was subjected to treatment with primary antibodies (anti-CD3 antibody (Abcam), anti-CD4 antibody (BD Biosciences), anti-CD8 antibody (BD Biosciences)) that were diluted at a ratio of 1:50, and reaction was allowed to proceed at 4°C overnight. The next day, the tissue was washed with PBS, and then allowed to react with a secondary antibody (Dako) at room temperature for 30 minutes. The tissue was washed again with PBS, allowed to react using the ABC kit (Dako), and then allowed to develop by addition of H₂O₂. Then, the tissue was subjected to dehydration, and then encapsulated.

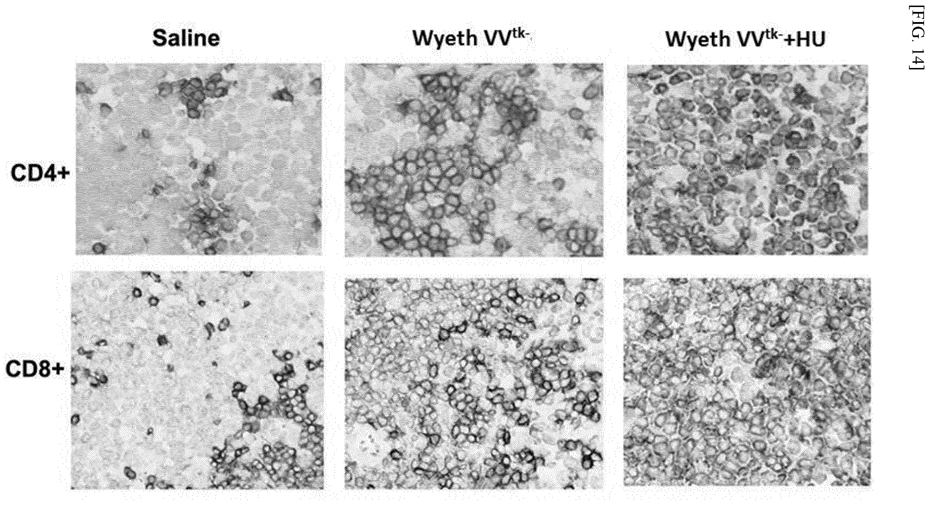

As a result, it was identified that CD4+ T cells and CD8+ T cells were distributed more abundantly in the tumor tissue of the mice of the experimental group (FIG. 14). From these results, it was identified that in a case where the recombinant vaccinia virus and hydroxyurea were co-administered, CD4+ T cells and CD8+ T cells in the spleen tissue were more differentiated and activated than a case where only the recombinant vaccinia virus was administered. That is, it was identified that in a case where the recombinant vaccinia virus and hydroxyurea were co-administered, adaptive immunity was better activated than a case where only the recombinant vaccinia virus was administered.

### Experimental Example 10. Identification of antigen-specific cytotoxic T lymphocyte (CTL) activation caused by recombinant vaccinia virus (OTS-412) and hydroxyurea in mouse breast cancer cell-transplanted mice: 4T1(I)

Balb/c mice (female, 7-week-old) purchased from ORIENT BIO (Busan, Korea) were subjected to a one-week acclimatization period, and then allografted with 4T1 cancer cell line (Korea Cell Line Bank) at 1×10⁶ cells. The tumor volume was observed until it reached 100 mm³ to 150 mm³, and then administration of a recombinant vaccinia virus was started. On the other hand, the Wyeth strain-derived recombinant vaccinia virus (OTS-412) hardly proliferates in a mouse breast cancer cell-transplanted mouse model. In addition, the breast cancer cell line-transplanted mouse is an animal model in which metastasis progresses throughout the body including lung tissue, and the metastasis is generally evaluated by the number of nodules on the tumor surface.

The produced mouse breast cancer cell-transplanted mice were divided into 4 groups (n=5). The group receiving intratumoral administration of saline was set as a negative control group, and the group receiving administration of the recombinant vaccinia virus (OTS-412, 1×10⁷ pfu) or hydroxyurea (30 mg/kg) were set as a positive control group. The group receiving administration of the recombinant vaccinia virus and hydroxyurea was set as an experimental group. The recombinant vaccinia virus was firstly intratumorally administered, and then secondly administered on day 7 after the first administration. The hydroxyurea was intraperitoneally administered once a day starting from 3 days before administration of the recombinant vaccinia virus to 3 days before sacrifice, except for the day of administration of the recombinant vaccinia virus.

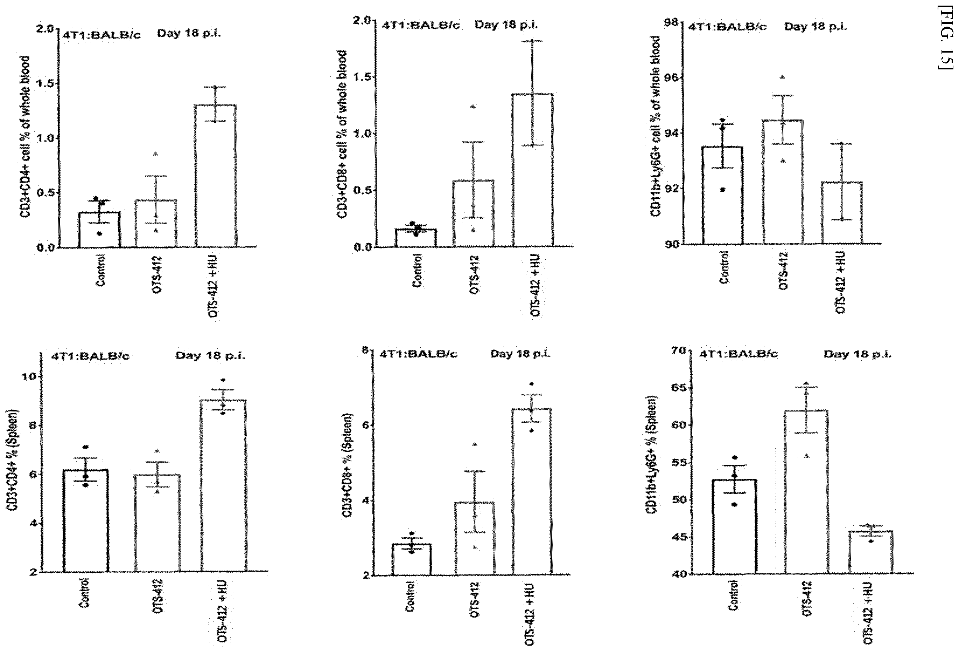

On day 18 after drug administration, the mice of each group were sacrificed, and the blood and spleen were collected therefrom. Distribution of immune cells in the blood and splenocytes was analyzed by flow cytometry. As a result, it was identified that distribution of CD4+ T cells and CD8+ T cells, which induce tumor immune responses, in the blood and spleen was highest in the mice of the experimental group. In addition, it was identified that the number of myeloid-derived suppressor cells (MDSCs) having an immunosuppressive function was remarkably low in the mice of the experimental group as compared with the mice of the negative control group and the positive control group (FIG. 15).

### Experimental Example 11. Identification of adaptive immunity increase effect of recombinant vaccinia virus (WR VV^{tk-}) and hydroxyurea in mouse breast cancer cell-transplanted mice: 4T1(II)

Balb/c mice (female, 10-week-old) purchased from ORIENT BIO (Busan, Korea) were subjected to a 2-day acclimatization period, and then subcutaneously transplanted in the left thigh with 4T1 cancer cell line (Korea Cell Line Bank) at 1×10⁶ cells. Two days later, the mice were subcutaneously transplanted in the right thigh with the same number of 4T1 cancer cell line. The tumor subcutaneously transplanted in the left thigh was observed until its volume reached 50 mm³ to 200 mm³, and then administration of a recombinant vaccinia virus was started.

The produced mouse breast cancer cell-transplanted mice were divided into 3 groups (n=6). The group receiving intratumoral administration of saline was set as a negative control group, and the group receiving administration of the recombinant vaccinia virus (WR VV^{tk-}, 1×10⁵ pfu) was set as a positive control group. In addition, the group receiving co-administration of the recombinant vaccinia virus and hydroxyurea (90 mg/kg) was set as an experimental group. The recombinant vaccinia virus was administered once into the left tumor, and the hydroxyurea was intraperitoneally administered 6 times per week starting from 1 day before administration of the recombinant vaccinia virus to day 14 after the administration, except for the day of administration of the recombinant vaccinia virus.

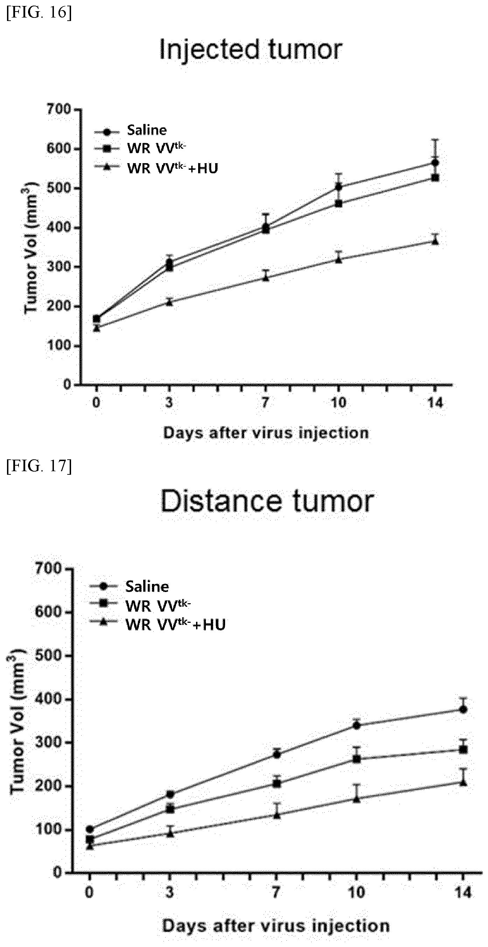

The volumes of the tumors subcutaneously transplanted in both thighs were measured on days 0, 3, 7, 10, and 14 after drug administration to the mice of each group. As a result, it was identified that the volume of the left tumor in the mice of the experimental group was suppressed by about 35% in growth as compared with the volume of the left tumor in the mice of the positive control group (FIG. 16). In addition, it was identified that the volume of the right tumor in the mice of the experimental group was suppressed by about 45% in growth as compared with the volume of the right tumor in the mice of the positive control group (FIG. 17). From these results, it was identified what effect co-administration of the recombinant vaccinia virus and hydroxyurea had on the surrounding tumor.

That is, it was identified that in a case where the tumor was locally treated by co-administration with the vaccinia virus and hydroxyurea, an anticancer effect was observed even in the tumor into which the virus had not been administered.

### Experimental Example 12. Identification of increased cancer selectivity upon co-administration of wild-type vaccinia virus (WR) and hydroxyurea in mouse renal cancer cell-transplanted mice (I)

Balb/c mice (female, 8-week-old) purchased from Orient Bio (Busan, Korea) were subjected to a one-week acclimatization period, and then allografted with Renca cancer cell line (Korea Cell Line Bank) at 5 × 10⁶ cells. The tumor volume was observed until it reached 100 mm³ to 150 mm³, and then administration of a wild-type Western Reserve strain vaccinia virus (WR) was started. Meanwhile, the wild-type Wyeth strain vaccinia virus has limited proliferative capacity in syngeneic mice.

The produced mouse renal cancer cell-transplanted mice were divided into 3 groups (n=8). The group receiving intraperitoneal administration of saline was set as a negative control group, and the group receiving administration of the wild-type vaccinia virus (WR, 1×10⁷ pfu) as a positive control group. In addition, the group receiving co-administration of the wild-type vaccinia virus and hydroxyurea (60 mg/kg) was set as an experimental group. The wild-type vaccinia virus was intratumorally administered twice; and the hydroxyurea was intraperitoneally administered 6 times per week starting from 1 day before administration of the wild-type vaccinia virus to day 21 after the administration, except for the day of administration of the wild-type vaccinia virus.

The mice of each group were sacrificed on day 22, and the tumors were isolated therefrom. Virus proliferation was compared through immunohistochemical staining using diaminobenzidine (DAB). Specifically, the tumor tissue was collected from the mice of each group. The tumor tissue was cut into 0.4 µm and dried. Subsequently, the tissue was washed with PBS, and then treated with bovine serum albumin (BSA). The tissue was subjected to treatment with a primary antibody (cat no. ABIN1606294, Antibodies-Online) that was diluted at a ratio of 1:50, and reaction was allowed to proceed at 4°C overnight. The next day, the tissue was washed with PBS, and then allowed to react with a secondary antibody (Alexa 594, cat no. A21205, Invitrogen) at room temperature for 30 minutes. The tissue was washed again with PBS, allowed to react using the ABC kit (Dako), and then allowed to develop by addition of H₂O₂. Then, the tissue was subjected to dehydration, and then encapsulated.

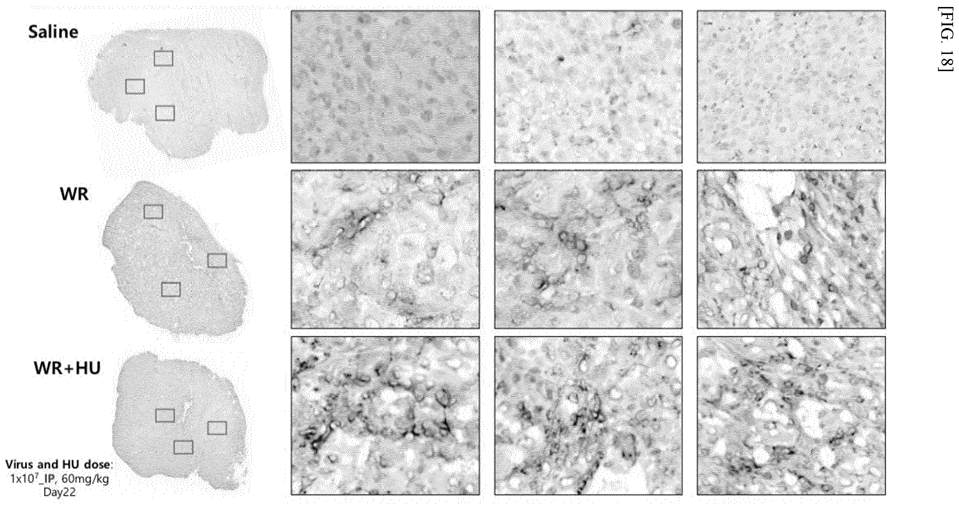

As a result, it was identified that the wild-type vaccinia virus was distributed more abundantly in the tumor tissue of the mice of the experimental group (FIG. 18). From these results, it was identified that more effective tumor-specific proliferation of the wild-type vaccinia virus was observed in a case where hydroxyurea was co-administered at the time of systemic administration of the wild-type vaccinia virus.

### Experimental Example 13. Identification of increased survival and cancer selectivity upon co-administration of wild-type vaccinia virus (WR) and hydroxyurea in normal mice (II)

Balb/c nu/nu mice (female, 7-week-old) purchased from ORIENT BIO (Busan, Korea) were subjected to a 2-day acclimatization period, and then administration of a wild-type Western Reserve strain vaccinia virus (WR) was started. On the other hand, the wild-type Wyeth strain vaccinia virus has limited proliferation capacity in syngeneic mice.

The mice were divided into two groups (n=12). The group receiving administration of the wild-type Western Reserve strain vaccinia virus (1×10⁷ pfu) was set as a control group, and the group receiving co-administration of the wild-type Western Reserve strain vaccinia virus and hydroxyurea (50 mg/kg) was set as an experimental group. The wild-type vaccinia virus was intranasally administered once; and the hydroxyurea was intraperitoneally administered 5 times per week starting from 1 day before administration of the wild-type vaccinia virus, except for the day of administration of the wild-type vaccinia virus.

On day 8, the mice of the control group and the experimental group were sacrificed, and the kidney and liver tissues were isolated therefrom. Immunohistochemical staining was performed. Paraffin blocks were created, and each block was deparaffinized using xylene and ethyl alcohol. The resulting block was subjected to antigen retrieval using a decloaking chamber. Then, a primary antibody (cat no. ABIN1606294, Antibodies-Online) was attached to this block and a FITC-labeled secondary antibody (Alexa 594, cat no. A21205, Invitrogen) was attached thereto. Then, observation was made using a fluorescence microscope.

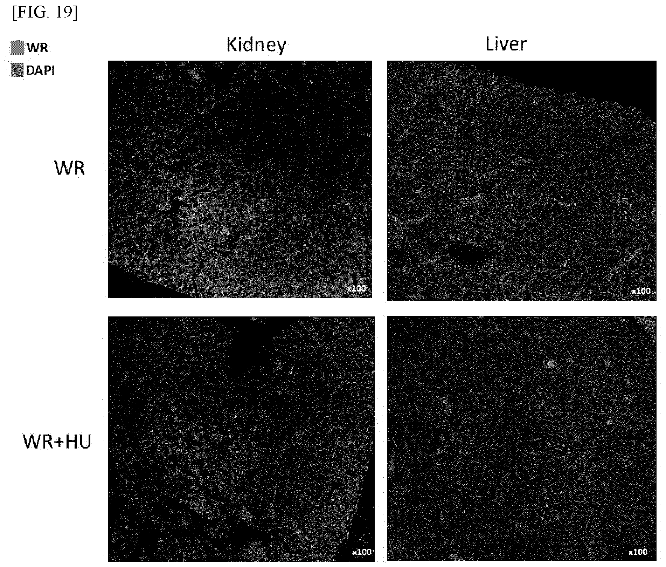

As a result, it was identified that the virus was distributed and proliferated in a small number in the liver and kidney tissues of the mice of the experimental group as compared with the liver and kidney tissues of the mice of the control group (FIG. 19).

### Experimental Example 14. Identification of absolute neutrophil count (ANC) in mouse renal cancer cell-transplanted mice upon co-administration of recombinant vaccinia virus (OTS-412) and hydroxyurea

Balb/c mice (female, 7-week-old) purchased from Orient Bio (Busan, Korea) were subjected to a 7-day acclimatization period, and then allografted with Renca cancer cell line (Korea Cell Line Bank) at 5 × 10⁶ cells. The tumor volume was observed until it reached 100 mm³ to 150 mm³, and then administration of a recombinant vaccinia virus was started. Meanwhile, the recombinant vaccinia virus (OTS-412) hardly proliferates in a mouse renal cancer cell-transplanted mice model.

The prepared mouse renal cancer cell-transplanted mice were divided into 4 groups (n = 4). The group receiving intratumoral administration of saline was set as a negative control group, and the group receiving administration of hydroxyurea (30 mg/kg) was set as a positive control group. The group receiving administration of the recombinant vaccinia virus (OTS-412, 1 × 10⁷ pfu), the group receiving administration of the recombinant vaccinia virus (OTS-412, 1 × 10⁷ pfu) and the recombinant human granulocyte colony-stimulating factor (rhG-CSF, 75 µg/kg), and the group receiving administration of the recombinant vaccinia virus (OTS-412, 1 × 10⁷ pfu) and hydroxyurea (30 mg/kg) were set as experimental groups. The recombinant vaccinia virus was administered intratumorally, and the second administration was performed 13 days after the first administration. The rhG-CSF or hydroxyurea was intraperitoneally administered from 2 days before the administration of the recombinant vaccinia virus until sacrificing the mice.

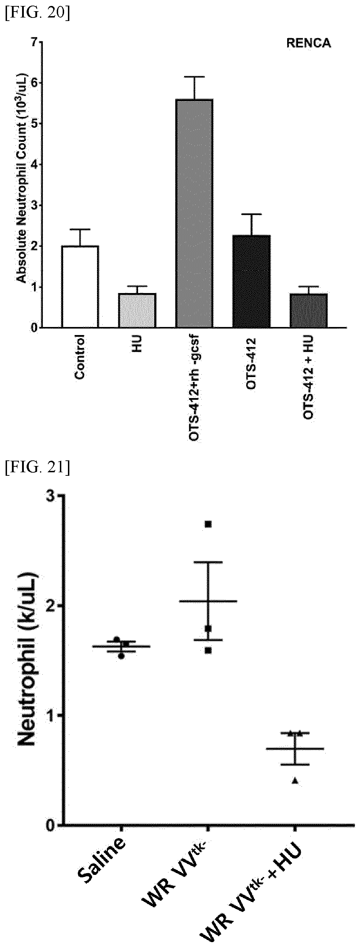

As a result of performing a complete blood count (CBC) by sacrificing 3 mice in each group on day 8 after administering the recombinant vaccinia virus, it was confirmed that the absolute neutrophil count (ANC) of the positive control group was decreased as compared with that of the negative control group. In the experimental groups, the absolute neutrophil count was measured to be similar to that of the positive control group only in the group in which the recombinant vaccinia virus and hydroxyurea were co-administered (FIG. 20).

### Experimental Example 15. Measurement of neutrophil count in mouse renal cancer cell-transplanted mice upon co-administration of recombinant vaccinia virus (WR VV^{tk-}) and hydroxyurea

Balb/c mice (female, 8-week-old) purchased from Orient Bio (Busan, Korea) were subjected to a one-week acclimatization period, and then allografted with Renca cancer cell line (Korea Cell Line Bank) at 5 × 10⁶ cells. The tumor volume was observed until it reached 100 mm³ to 150 mm³, and then administration of a recombinant vaccinia virus was started. Meanwhile, the recombinant vaccinia virus (WR VV^{tk-}) can proliferate in a mouse renal cancer cell-transplanted mice model.

The prepared mouse renal cancer cell-transplanted mice were divided into 3 groups (n = 8). The group receiving intratumoral administration of saline was set as a control group. The group receiving administration of the recombinant vaccinia virus (WR VV^{tk-}, 1 × 10⁷ pfu) and the group receiving co-administration of the recombinant recombinant vaccinia virus (WR VV^{tk-}, 1 × 10⁷ pfu) and hydroxyurea (60 mg/kg) were set as experimental groups. The recombinant vaccinia virus was administered twice intraperitoneally, and hydroxyurea was administered 6 times per week intraperitoneally from 1 day before the administration of the recombinant vaccinia virus to day 21 after the administration, except for the day of administering the recombinant vaccinia virus.

As a result of performing a complete blood count (CBC) after sacrificing 3 mice in each group on day 8 after administering the recombinant vaccinia virus, the neutrophil count in the blood of the mice in the group receiving administration of only the recombinant vaccinia virus was increased as compared with the control group. Meanwhile, the neutrophil count in the blood of the mice in the group co-administered with the recombinant vaccinia virus and hydroxyurea was significantly reduced as compared with the control group (FIG. 21).

### Experimental Example 16. Identification of absolute neutrophil count (ANC) in mouse renal cancer cell-transplanted mice upon co-administration of recombinant vaccinia virus (WOTS-418) and hydroxyurea

Balb/c mice (female, 8-week-old) purchased from Orient Bio (Busan, Korea) were subjected to a one-week acclimatization period, and then allografted with Renca cancer cell line (Korea Cell Line Bank) at 5 × 10⁶ cells. The tumor volume was observed until it reached 100 mm³ to 150 mm³, and then administration of a Western Reserve strain vaccinia virus-derived oncolytic virus (WOTS-418) was started.

The prepared mouse renal cancer cell-transplanted mice were divided into 3 groups (n = 3). The group receiving intraperitoneal administration of saline was set as a negative control group, the group receiving administration of oncolytic virus (WOTS-418, 1 × 10⁷ pfu) was set as a positive control group, and the group receiving co-administration of the oncolytic virus and hydroxyurea (30 mg/kg) was set as an experimental group. The oncolytic virus was administered once intraperitoneally, and hydroxyurea was administered intraperitoneally daily from 1 day before the administration of the oncolytic virus to day 2 after the administration.

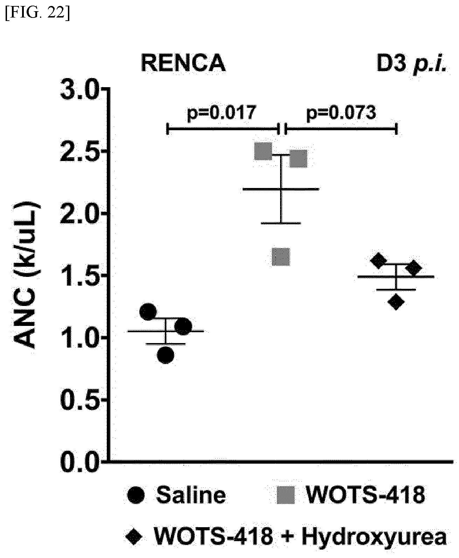

As a result of performing a complete blood count (CBC) by sacrificing the mice in each group on day 3 after administering the oncolytic virus, it was confirmed that there was a tendency of a decrease in the neutrophil count of the experimental group (FIG. 22).

### II. Identification of synergistic anticancer effect by co-administration of vaccinia virus and lenalidomide

### Experimental Example 17. Identification of absolute neutrophil count (ANC) in mouse renal cancer cell-transplanted mice upon administration of lenalidomide

Balb/c mice (female, 8-week-old) purchased from Orient Bio (Busan, Korea) were subjected to a one-week acclimatization period, and then allografted with Renca cancer cell line (Korea Cell Line Bank) at 5 × 10⁶ cells. After another one-week acclimatization period, the mice were divided into 3 groups (n = 3) and administered intraperitoneally with saline, hydroxyurea (30 mg/kg), and lenalidomide (30 mg/kg), respectively, daily from 1 day before the administration of the oncolytic virus to day 4 after the administration.

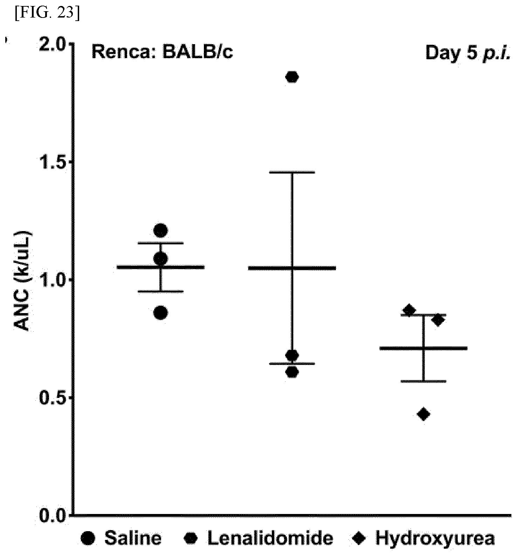

As a result of performing a complete blood count (CBC) by sacrificing the mice in each group on day 5 after administering the oncolytic virus, it was confirmed that the neutrophil count of the mice in the group administered with lenalidomide was reduced as compared with the group administered with saline, except for one outlier mouse. In addition, it was confirmed that the neutrophil count of the mice of the group administered with hydroxyurea was significantly lower than that of the group administered with saline (FIG. 23).

### Experimental Example 18. Identification of absolute neutrophil count (ANC) in mouse renal cancer cell-transplanted mice upon co-administration of recombinant vaccinia virus (WOTS-418) and lenalidomide

Balb/c mice (female, 8-week-old) purchased from Orient Bio (Busan, Korea) were subjected to a one-week acclimatization period, and then allografted with Renca cancer cell line (Korea Cell Line Bank) at 5 × 10⁶ cells. The tumor volume was observed until it reached 100 mm³ to 150 mm³, and then administration of a Western Reserve strain vaccinia virus-derived anticancer (WOTS-418) was started.

The prepared mouse renal cancer cell-transplanted mice were divided into 4 groups (n = 5). The group receiving intraperitoneal administration of saline was set as a negative control group, the group receiving administration of oncolytic virus (WOTS-418, 1 × 10⁷ pfu) was set as a positive control group, and the group receiving co-administration of the oncolytic virus and hydroxyurea (30 mg/kg) and the group receiving co-administration of the oncolytic virus and lenalidomide (30 mg/kg) were set as experimental groups. The oncolytic virus was administered once intraperitoneally, and hydroxyurea or lenalidomide was administered intraperitoneally daily from 1 day before the administration of the oncolytic virus to day 2 after the administration.

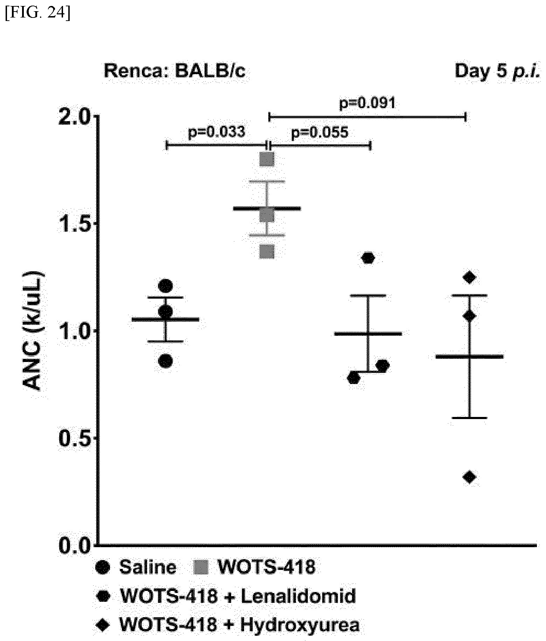

As a result of performing a complete blood count (CBC) by sacrificing the mice in each group on day 5 after administering the oncolytic virus, it was confirmed that the neutrophil count of the mice of the experimental groups was significantly lower than that of the positive control group administered with the oncolytic virus alone (FIG. 24).

### Experimental Example 19. Identification of cancer therapeutic effect upon co-administration of recombinant vaccinia virus (WR VV^{TK-}) and lenalidomide in mouse renal cancer cell-transplanted mice

Balb/c mice (female, 8-week-old) purchased from Orient Bio (Busan, Korea) were subjected to a one-week acclimatization period, and then allografted with Renca cancer cell line (Korea Cell Line Bank) at 5 × 10⁶ cells. The tumor volume was observed until it reached 100 mm³ to 150 mm³, and then administration of an oncolytic virus was started. Meanwhile, the Western Reserve strain vaccinia virus-derived oncolytic virus (WR VV^{tk-}) can proliferate in a mouse renal cancer cell-transplanted mice model.

The prepared mouse renal cancer cell-transplanted mice were divided into 6 groups (n = 8). Experiments were performed by dividing the mice as follows: the group receiving intraperitoneal administration of saline was set as a control group, and the group receiving administration of the oncolytic virus (WR VV^{tk-}, 1×10⁷ pfu) alone, the group receiving co-administration of the oncolytic virus and hydroxyurea (60 mg/kg), and the group receiving co-administration of the oncolytic virus and lenalidomide (25 mg/kg). The oncolytic virus was administered twice intraperitoneally, and hydroxyurea or lenalidomide was administered 6 times per week intraperitoneally 1 day before the administration of the oncolytic virus to day 21 after the administration, except for the day of administering the oncolytic virus.

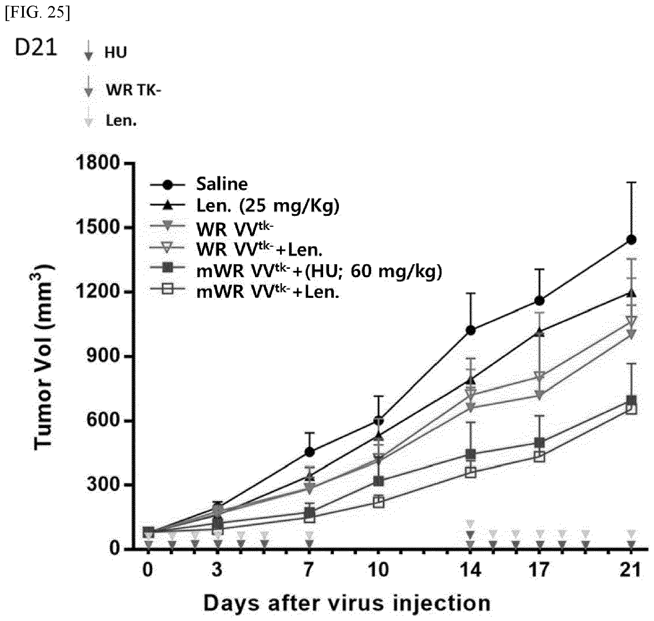

As a result of measuring the tumor voume by sacrificing mice of each group on day 21, it was confirmed that the tumor growth was significantly inhibited in the group administered with the oncolytic virus alone as compared with the control group (p<0.001). The group co-administered with oncolytic virus and lenalidomide showed a tendency to inhibit tumor growth as compared with the group treated with lenalidomide alone. In particular, it was observed that the group co-administered with the oncolytic virus and hydroxyurea significantly inhibited tumor growth as compared with the group treated with the oncolytic virus alone (p<0.05), and it was confirmed that the group co-administered with lenalidomide and hydroxyurea showed higher inhibition of tumor growth than the group co-administered with the oncolytic virus and hydroxyurea (FIG. 25).

### III. Identification of synergistic anticancer effect by co-administration of vaccinia virus and palbociclib

### Experimental Example 20. Identification of cancer therapeutic effect upon co-administration of recombinant vaccinia virus (WR VV^{TK-}) and palbociclib in mouse renal cancer cell-transplanted mice

### Experimental Example 20.1. Preparation of mouse renal cancer cell-transplanted mice and drug administration

Balb/c mice (female, 8-week-old) purchased from Orient Bio (Busan, Korea) were subjected to a one-week acclimatization period, and then allografted with Renca cancer cell line (Korea Cell Line Bank) at 5 × 10⁶ cells. The tumor volume was observed until it reached 100 mm³ to 150 mm³, and then administration of an oncolytic virus was started. Meanwhile, a Western Reserve strain vaccinia virus-derived oncolytic virus (WOTS-418) can proliferate in a mouse renal cancer cell-transplanted mice model.

The prepared mouse renal cancer cell-transplanted mice were divided into 5 groups (n = 5). Experiments were performed by dividing the mice as follows: the group receiving intraperitoneal administration of saline was set as a control group, and the group receiving administration of the oncolytic virus (WR VV^{tk-}, 1 × 10⁷ pfu) alone, the group receiving co-administration of the oncolytic virus and hydroxyurea (60 mg/kg), and the group receiving co-administration of the oncolytic virus and palbociclib (50 mg/kg or 100 mg/kg). The oncolytic virus was administered once intraperitoneally, and palbociclib was orally administered once per week from 5 days before the administration of oncolytic virus, and hydroxyurea was administered 6 times per week intraperitoneally from 1 day before the administration of the oncolytic virus to day 19 after the administration, except for the day of administering the oncolytic virus.

### Experimental Example 20.2. Identification of changes in tumor volume

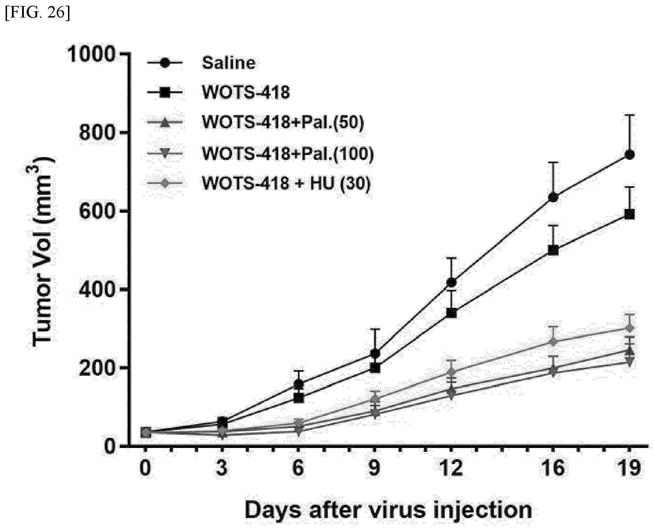

As a result of measuring the tumor volume by sacrificing mice in each group of Experimental Example 20.1 on day 19, it was confirmed that tumor growth was significantly inhibited statistically in the group administered with the oncolytic virus alone as compared with the control group (p<0.05). It was confirmed that the group co-administered with the oncolytic virus and hydroxyurea or palbociclib significantly inhibited tumor growth as compared with the group administered with the oncolytic virus alone (p<0.0001) (FIG. 26).

### Experimental Example 20.3. Identification of changes in body weight

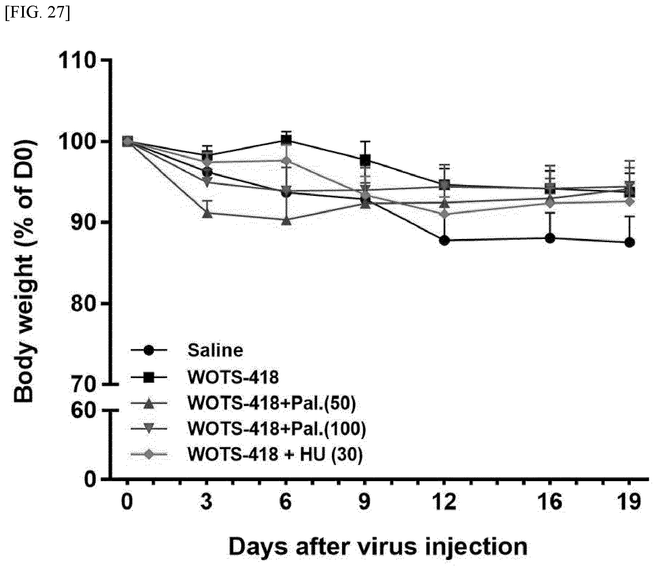

After administration of each drug to the control group and each group of Experimental Example 20.1, the weight of mice was measured on days 3, 6, 9, 12, 16, and 19. As a result, there was no tendency of a decrease in body weight in all of the co-administration groups, and even though there was a tendency of a continuous decrease in body weight in the group administered with the oncolytic virus alone, the body weight on day 19 was maintained close to 90% as compared with the body weight at the start of administration, thus confirming that the safety was not at a level of concern (FIG. 27).

### IV. Identification of synergistic anticancer effect by co-administration of vaccinia virus, granulopoiesis inhibitor (hydroxyurea), and immune checkpoint inhibitor

### Experimental Example 21. Identification of cancer therapeutic effect of co-administration of oncolytic virus (Wyeth VV^{tk-}), PD-1 inhibitor, and hydroxyurea in mouse renal cancer cell-transplanted mice: Renca (I)

In order to confirm the additional effect of administering hydroxyurea when an oncolytic virus and a PD-1 inhibitor (CD279, BioXCell) *(i.e.,* one of the immune checkpoint inhibitors) are co-administered, an experiment was performed using mouse renal cancer cell-transplanted mice.

First, Balb/c mice (female, 8-week-old) purchased from Orient Bio (Busan, Korea) were subjected to a 7-day acclimatization period, and then allografted with Renca cancer cell line (Korea Cell Line Bank) at 5 × 10⁶ cells. The tumor volume was observed until it reached 200 mm³ to 300 mm³, and then administration of an oncolytic virus (Wyeth VV^{tk-}) was started. The oncolytic virus has limited proliferative capacity in an allograft model.

The prepared mouse renal cancer cell-transplanted mice were divided into 5 groups (n = 5). The group receiving intraperitoneal administration of saline was set as a negative control group, and the group receiving administration of a mouse PD-1 inhibitor (200 µg/mouse), the group receiving intratumoral administration of the oncolytic virus (Wyeth VV^{tk-}, 1 × 10⁷ pfu), and the group receiving co-administration of the oncolytic virus (Wyeth VV^{tk-}, 1 × 10⁷ pfu) and a PD-1 inhibitor were set as positive control groups. In addition, the group receiving co-administration of the oncolytic virus (Wyeth VV^{tk-}, 1 × 10⁷ pfu), a PD-1 inhibitor, and hydroxyurea (30 mg/kg) was set as an experimental group. In this case, the oncolytic virus was administered once intratumorally, the PD-1 inhibitor was administered intraperitoneally once every two days on days 14, 16, 18, and 20, and hydroxyurea was administered 6 times per week intraperitoneally.

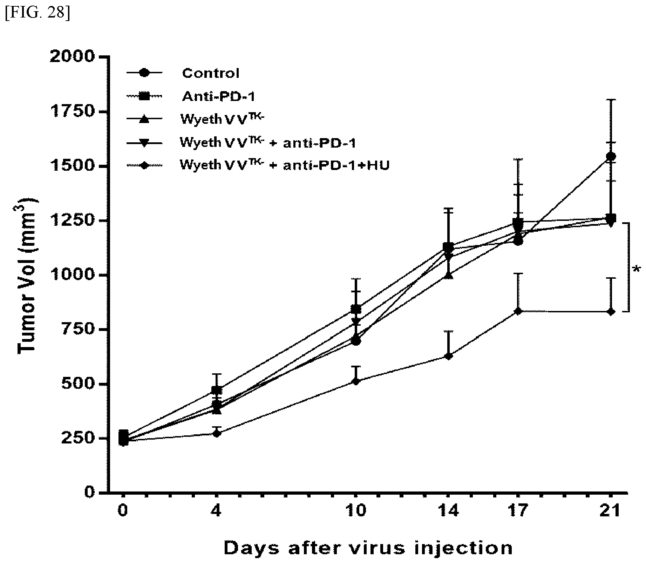

The tumor volume was measured on days 0, 4, 10, 14, 17, and 21 after the administration of the drugs to the mice of each group. As a result, it was confirmed that the tumor volume of the mice in the experimental group was significantly inhibited as compared with the tumor volume of the mice in the positive control group (FIG. 28).

### Experimental Example 22. Identification of cancer therapeutic effect of co-administration of oncolytic virus (Wyeth VV^{tk-}), CTLA4 inhibitor, and hydroxyurea in mouse renal cancer cell-transplanted mice: Renca (II)

In order to confirm the additional effect of administering hydroxyurea when an oncolytic virus and a CTLA-4 inhibitor (B7-H1, BioXCell)) *(i.e.,* immune checkpoint inhibitor) are co-administered, an experiment was performed using mouse renal cancer cell-transplanted mice.

First, Balb/c mice (female, 8-week-old) purchased from Orient Bio (Busan, Korea) were subjected to a 7-day acclimatization period, and then allografted with Renca cancer cell line (Korea Cell Line Bank) at 5 × 10⁶ cells. The tumor volume was observed until it reached 50 mm³ to 150 mm³, and then administration of an oncolytic virus (Wyeth VV^{tk-}) was started. The oncolytic virus has limited proliferative capacity in an allograft model.

The prepared mouse renal cancer cell-transplanted mice were divided into 5 groups (n = 6). The group receiving intraperitoneal administration of saline was set as a negative control group, and the group receiving administration of a CTLA-4 inhibitor (150 µg/mouse), the group receiving intratumoral administration of the oncolytic virus (Wyeth VV^{tk-}, 1 × 10⁷ pfu), and the group receiving co-administration of the oncolytic virus (Wyeth VV^{tk-}, 1 × 10⁷ pfu) and a CTLA-4 inhibitor were set as positive control groups. In addition, the group receiving co-administration of the oncolytic virus (Wyeth VV^{tk-}, 1 × 10⁷ pfu), a CTLA-4 inhibitor, and hydroxyurea (30 mg/kg) was set as an experimental group. In this case, the oncolytic virus was administered once intratumorally, the CTLA-4 inhibitor was administered intraperitoneally once every two days on days 3, 5, 7, and 9, and hydroxyurea was administered 6 times per week intraperitoneally.

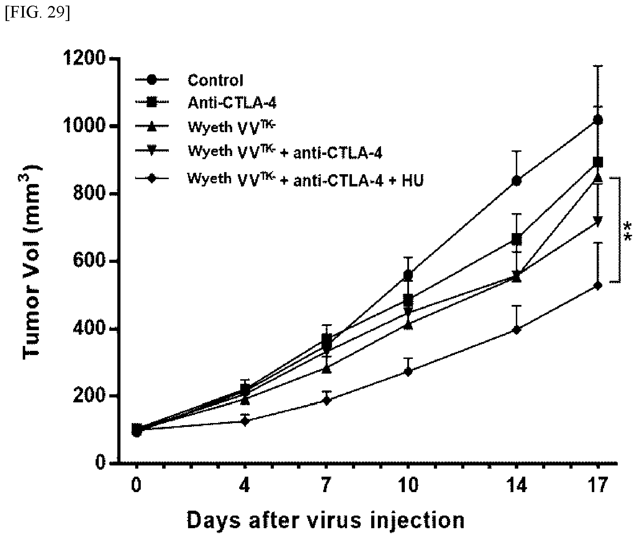

The tumor volume was measured on days 0, 4, 7, 10, 14, and 17 after the administration of the drugs to the mice of each group. As a result, it was confirmed that the tumor volume of the mice in the experimental group was significantly inhibited as compared with the tumor volume of the mice in the positive control group (FIG. 29). From these results, it was confirmed that when co-administering an oncolytic virus and an immune checkpoint inhibitor (CTLA-4 inhibitor), an additional administration of hydroxyurea thereto resulted in exhibition of an excellent effect of inhibiting mouse renal cancer.

### Experimental Example 23. Identification of cancer therapeutic effect of co-administration of oncolytic virus (Wyeth VV^{tk-}), PD-L1 inhibitor, and hydroxyurea in mouse renal cancer cell-transplanted mice: Renca (III)

In order to confirm the additional effect of administering hydroxyurea when an oncolytic virus and a PD-L1 inhibitor (CD152, BioXCell) *(i.e.,* one of the immune checkpoint inhibitors) are co-administered, an experiment was performed using mouse renal cancer cell-transplanted mice.

First, Balb/c mice (female, 8-week-old) purchased from Orient Bio (Busan, Korea) were subjected to a 7-day acclimatization period, and then allografted with Renca cancer cell line (Korea Cell Line Bank) at 5 × 10⁶ cells. The tumor volume was observed until it reached 50 mm³ to 100 mm³, and then administration of an oncolytic virus (Wyeth VV^{tk-}) was started. The oncolytic virus has limited proliferative capacity in allograft model.

The prepared mouse renal cancer cell-transplanted mice were divided into 5 groups (n = 6). The group receiving intraperitoneal administration of saline was set as a negative control group, and the group receiving administration of a PD-L 1 inhibitor (300 µg/mouse), the group receiving intratumoral administration of the oncolytic virus (Wyeth VV^{tk-}, 1 × 10⁷ pfu), and the group receiving co-administration of the oncolytic virus (Wyeth VV^{tk-}, 1 × 10⁷ pfu) and a PD-L1 inhibitor were set as positive control groups. In addition, the group receiving co-administration of the oncolytic virus (Wyeth VV^{tk-}, 1 × 10⁷ pfu), a PD-L1 inhibitor, and hydroxyurea (30 mg/kg) was set as an experimental group. In this case, the oncolytic virus was administered once intratumorally, the PD-L1 inhibitor was administered intraperitoneally on days 0, 3, 7, 10, 14, 17, and 21, and hydroxyurea was administered 6 times per week intraperitoneally.

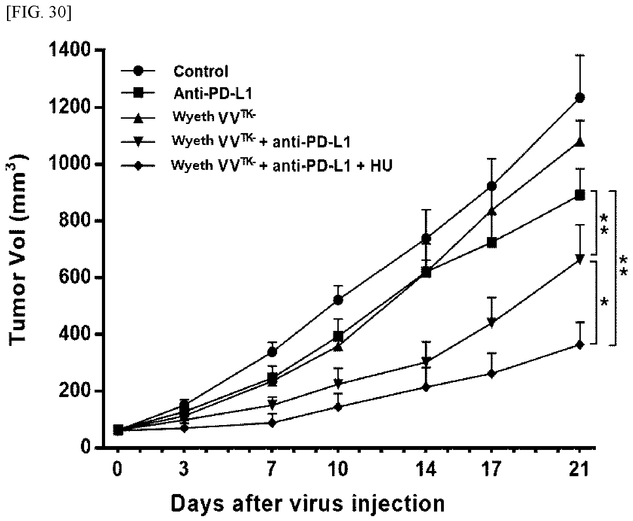

The tumor volume was measured on days 0, 3, 7, 10, 14, 17, and 21 after the administration of the drugs to the mice of each group. As a result, it was confirmed that the tumor volume of the mice in the experimental group was significantly inhibited as compared with the tumor volume of the mice in the positive control group (FIG. 30). In particular, comparing the tumor volume before sacrificing the mice, it was confirmed that the tumor volume of the experimental group was about 46% smaller than that of the group receiving the co-administration of the oncolytic virus and the PD-L1 inhibitor.

From these results, it was confirmed that when co-administering an oncolytic virus and an immune checkpoint inhibitor (PD-L1 inhibitor), an additional administration of hydroxyurea thereto resulted in exhibition of an excellent effect of inhibiting mouse renal cancer.

### Experimental Example 24. Identification of cancer therapeutic effect of oncolytic virus (WR VV^{tk-}), CTLA-4 inhibitor, and hydroxyurea in mouse breast cancer cell-transplanted mice: 4T1 (I)

### Experimental Example 24.1. Preparation of mouse breast cancer cell-transplanted mice and drug administration

In order to confirm the additional effect of administering hydroxyurea when an oncolytic virus and a CTLA-4 inhibitor (B7-H1, BioXCell) are co-administered, an experiment was performed using mouse breast cancer cell-transplanted mice.

First, Balb/c mice (female, 8-week-old) purchased from Orient Bio (Busan, Korea) were subjected to a 7-day acclimatization period, and then allografted with 4T1 cancer cell line (Korea Cell Line Bank) at 1 × 10⁶ cells. The tumor volume was observed until it reached 50 mm³ to 150 mm³, and then administration of an oncolytic virus (WR VV^{tk-}) was started. The Western Reserve strain vaccinia virus-derived oncolytic virus (WR VV^{tk-}) has a stronger proliferative capacity in an allograft model than the Wyeth strain vaccinia virus-derived oncolytic virus.

The prepared mouse breast cancer cell-transplanted mice were divided into 5 groups (n = 5). The group receiving intraperitoneal administration of saline was set as a negative control group, and the group receiving administration of a CTLA-4 inhibitor (300 µg/mouse), the group receiving intratumoral administration of the oncolytic virus (WR VV^{tk-}, 1 × 10⁷ pfu), and the group receiving co-administration of the oncolytic virus (Wyeth VV^{tk-}, 1 × 10⁷ pfu) and a CTLA-4 inhibitor were set as positive control groups. In addition, the group receiving co-administration of the oncolytic virus (WR VV^{tk-}, 1 × 10⁷ pfu), a CTLA-4 inhibitor, and hydroxyurea (30 mg/kg) was set as an experimental group. In this case, the oncolytic virus was administered twice intraperitoneally, the CTLA-4 inhibitor was administered intraperitoneally on days 3, 5, 7, and 9, and hydroxyurea was administered 6 times per week intraperitoneally.

### Experimental Example 24.2. Identification of changes in tumor volume

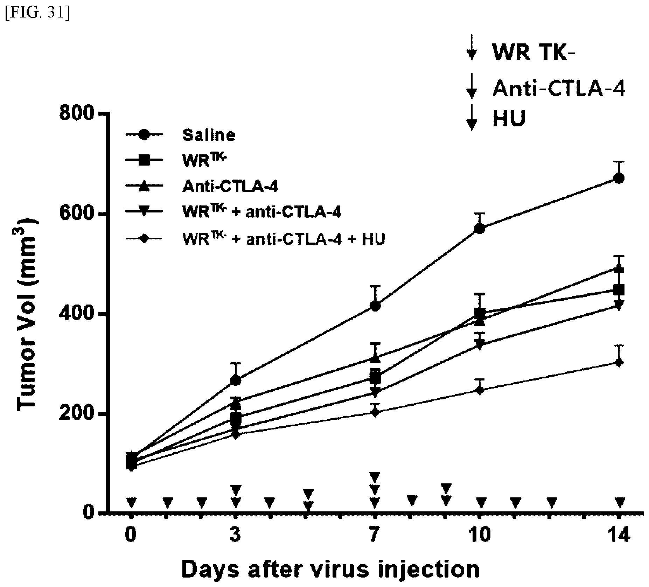

The tumor volume was measured on days 0, 3, 7, 10, and 14 after the administration of a drug to the mice of each group. As a result, it was confirmed that the tumor volume of the mice of the experimental group was significantly inhibited as compared with the tumor volume of the mice of the positive control group (FIG. 31).

### Experimental Example 24.3. Analysis of survival rate

Additionally, for the survival period, it was confirmed that the survival period and survival rate of mice in the experimental group were shown to be the best. From this result, it was confirmed that when the oncolytic virus and the CTLA-4 inhibitor were co-administered in mouse breast cancer cell-transplanted mice, additional administration of hydroxyurea showed a significant effect.

### Experimental Example 25. Identification of cancer therapeutic effect of oncolytic virus (WOTS-418), PD-L1 inhibitor, and hydroxyurea in mouse breast cancer cell-transplanted mice: 4T1 (II)

### Experimental Example 25.1. Preparation of mouse breast cancer cell-transplanted mice and drug administration

In order to confirm the additional effect of administering hydroxyurea when an oncolytic virus and a PD-L1 inhibitor (CD152, BioXCell) are co-administered, an experiment was performed using mouse breast cancer cell-transplanted mice.

First, Balb/c mice (female, 8-week-old) purchased from Orient Bio (Busan, Korea) were subjected to a 7-day acclimatization period, and then allografted with 4T1 cancer cell line (Korea Cell Line Bank) at 1 × 10⁶ cells. The tumor volume was observed until it reached 50 mm³ to 100 mm³, and then administration of an oncolytic virus (WOTS-418) was started. The Western Reserve strain has a stronger proliferative capacity in an allograft model than the Wyeth strain.

The prepared mouse breast cancer cell-transplanted mice were divided into 5 groups (n = 6). The group receiving intraperitoneal administration of saline was set as a negative control group, and the group receiving administration of a PD-L 1 inhibitor (300 µg/mouse), the group receiving intratumoral administration of the oncolytic virus (WOTS-418, 1 × 10⁷ pfu), and the group receiving co-administration of the oncolytic virus (WOTS-418, 1× 10⁷ pfu) and a PD-L1 inhibitor were set as positive control groups. In addition, the group receiving co-administration of the oncolytic virus (WOTS-418, 1×10⁷ pfu), a PD-L1 inhibitor, and hydroxyurea (30 mg/kg) was set as an experimental group. In this case, the oncolytic virus was administered twice intraperitoneally, the PD-L1 inhibitor was administered intraperitoneally on days 3, 5, 7, and 9, and hydroxyurea was administered 6 times per week intraperitoneally.

### Experimental Example 25.2. Identification of changes in tumor volume

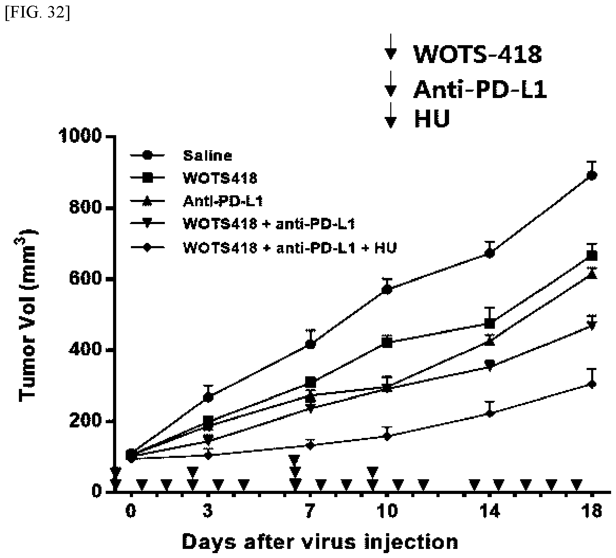

The tumor volume was measured on days 0, 3, 7, 10, and 14 after the administration of the drugs to the mice of each group of Experimental Example 5.1. As a result, it was confirmed that the tumor volume of the mice in the experimental group was significantly inhibited as compared with the tumor volume of the mice in the positive control group (FIG. 32). In particular, comparing the tumor volume before sacrificing the mice, it was confirmed that the tumor volume of the experimental group was about 30% smaller than that of the group receiving the co-administration of the oncolytic virus and the PD-L1 inhibitor.

From these results, it was confirmed that when co-administering an oncolytic virus and an immune checkpoint inhibitor (PD-L1 inhibitor), an additional administration of hydroxyurea thereto resulted in exhibition of a synergistic effect of inhibiting mouse breast cancer.

### Experimental Example 25.3. Analysis of survival rate

The survival rate for 30 days of mice in each group of Experimental Example 5.1 was analyzed. As a result, it was confirmed that the survival rate of mice in the experimental group was higher than that of the mice in the negative and positive control groups.

### Experimental Example 26. Analysis of survival rate by oncolytic virus (WR, WOTS-418), PD-L1 inhibitor, and hydroxyurea in mouse colorectal cancer cell-transplanted mice: CT-26 I

In order to confirm the safety upon co-administration of Western Reserve strain vaccinia virus (WR), a PD-L1 inhibitor, and hydroxyurea, the survival period was analyzed using mouse colorectal cancer cell-transplanted mice.

First, Balb/c mice (female, 8-week-old) purchased from Orient Bio (Busan, Korea) were subjected to a 7-day acclimatization period, and then subcutaneously transplanted with colorectal cancer (CT-26) (Korea Cell Line Bank) at 1 × 10⁶ cells. After 7 days, oncolytic virus (WR) and a PD-L1 inhibitor were administered intraperitoneally, and hydroxyurea was administered daily for 5 days from the following day. Meanwhile, the Western Reserve strain vaccinia virus has a stronger proliferative capacity in an allograft model than the Wyeth strain vaccinia virus.

The prepared mouse colorectal cancer cell-transplanted mice were divided into 5 groups (n = 13). The group receiving intraperitoneal administration of saline was set as a negative control group, and the group receiving administration of a PD-L 1 inhibitor (300 µg/mouse) alone and the group receiving co-administration of the oncolytic virus (WOTS-418) and hydroxyurea (30 mg/kg) were set as positive control groups. In addition, the group receiving co-administration of the oncolytic virus (WR, 1 × 10⁶ pfu or WOTS-418, 1 × 10⁷ pfu), a PD-L1 inhibitor, and hydroxyurea was set as an experimental group. In this case, the oncolytic virus was administered once intraperitoneally, the PD-L1 inhibitor was administered intraperitoneally on days 1, 4, 8, and 11, and hydroxyurea was administered 5 times per week intraperitoneally.

As a result of analyzing the survival curves of mice in each group, it was observed that the survival period of the mice in the experimental group was the longest as compared with the mice in the negative control group and the mice in the positive control group. From these results, it was confirmed that the safety was improved when the oncolytic virus, immune checkpoint inhibitor and hydroxyurea were co-administered.

### Experimental Example 27. Analysis of survival rate by Western Reserve strain vaccinia virus (WR), CTLA-4 inhibitor, and hydroxyurea in mouse renal cancer cell-transplanted mice: Renca (IV)

In order to confirm the additional effect of administering hydroxyurea when Western Reserve strain vaccinia virus (WR) and a CTLA-4 inhibitor (B7-H1, BioXCell) *(i.e.,* one of the immune checkpoint inhibitors) are co-administered, an experiment was performed using mouse renal cancer cell-transplanted mice.

First, Balb/c mice (female, 8-week-old) purchased from Orient Bio (Busan, Korea) were subjected to a 7-day acclimatization period, and then allografted with Renca cancer cell line (Korea Cell Line Bank) at 5 × 10⁶ cells. The tumor volume was observed until it reached 30 mm³ to 50 mm³, and then administration of Western Reserve strain vaccinia virus (WR) was started. The Western Reserve strain vaccinia virus (WR) has a stronger proliferative capacity in an allograft model than the Wyeth strain vaccinia virus.

The prepared mouse renal cancer cell-transplanted mice were divided into 4 groups (n = 4). The group receiving intraperitoneal administration of saline was set as a negative control group, and the group receiving co-administration of the Western Reserve strain vaccinia virus (WR, 1 × 10⁵ pfu) and hydroxyurea (30 mg/kg) and the group receiving co-administration of the Western Reserve strain vaccinia virus and a CTLA-4 inhibitor (150 µg/mouse) were set as positive control groups. In addition, the group receiving co-administration of the Western Reserve strain vaccinia virus, a CTLA-4 inhibitor, and hydroxyurea was set as an experimental group. In this case, the Western Reserve strain vaccinia virus was administered once intraperitoneally, the CTLA-4 inhibitor was administered intraperitoneally on days 2, 4, 6, and 8, and hydroxyurea was administered 4 times per week intraperitoneally.

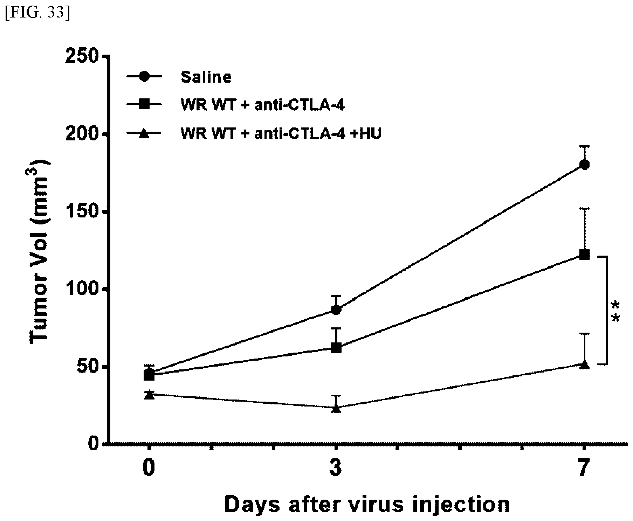

The tumor volume was measured on days 0, 3, and 7 after the administration of the drugs to the mice of each group. As a result, it was confirmed that the tumor volume of the mice of the experimental group was significantly inhibited as compared with the tumor volume of the mice of the positive control group (FIG. 33).

## Claims

1. A pharmaceutical composition for treating cancer, comprising as active ingredients:
a vaccinia virus; and
a granulopoiesis inhibitor.

2. The pharmaceutical composition of claim 1, wherein the vaccinia virus belongs to Western Reserve (WR), New York Vaccinia Virus (NYVAC), Wyeth ( New York City Board of Health; NYCBOH), an LC16m8, Lister, Copenhagen, Tian Tan, USSR, TashKent, Evans, International Health Division-J (IHD-J), or International Health Division-White (IHD-W) vaccinia virus strain.

3. The pharmaceutical composition of claim 1, wherein the vaccinia virus is a wild-type vaccinia virus or a recombinant vaccinia virus.

4. The pharmaceutical composition of claim 3, wherein the recombinant vaccinia virus is obtained by deleting at least one gene from a wild-type vaccinia virus or inserting a foreign gene thereinto.

5. The pharmaceutical composition of claim 4, wherein the gene of the wild-type vaccinia virus is any one selected from the group consisting of thymidine kinase gene, vaccinia growth factor gene, F13.5L gene, F14.5L gene, A56R gene, B18R gene, and a combination thereof.

6. The pharmaceutical composition of claim 4, wherein the foreign gene is a gene encoding any one selected from the group consisting of herpes simplex virus thymidine kinase (HSV-TK), mutated HSV-TK, granulocyte-macrophage colony-stimulating factor (GM-CSF), cytosin deaminase (CD), carboxylesterase type 1, carboxylesterase type 2, interferone beta (lNF-β), somatostatin receptor 2, and a combination thereof.

7. The pharmaceutical composition of claim 1, wherein the cancer is any one selected from the group consisting of lung cancer, colorectal cancer, prostate cancer, thyroid cancer, breast cancer, brain cancer, head and neck cancer, esophageal cancer, skin cancer, thymic cancer, gastric cancer, colon cancer, liver cancer, ovarian cancer, uterine cancer, bladder cancer, rectal cancer, gallbladder cancer, biliary tract cancer, pancreatic cancer, and a combination thereof.

8. The pharmaceutical composition of claim 1, wherein the granulopoiesis inhibitor is hydroxyurea, lenalidomide, thalidomide, tadalafil, palbociclib, alkylating agents, anthracyclines, antimetabolites, camptothecins, epipodophyllotoxins, mitomycin C, taxanes, or vinblastine.

9. The pharmaceutical composition of claim 1, wherein the granulocyte is a neutrophil.

10. The pharmaceutical composition of claim 1, further comprising an immune checkpoint inhibitor.

11. The pharmaceutical composition of claim 1, wherein the cancer selectivity of the vaccinia virus is increased.

12. A kit for preventing or treating cancer comprising:
a first composition that comprises a vaccinia virus as an active ingredient; and
a second composition that comprises a granulopoiesis inhibitor as an active ingredient.

13. The kit of claim 12, further comprising a third composition that comprises an immune checkpoint inhibitor as an active ingredient.

14. A method for treating cancer, comprising:
administering a granulopoiesis inhibitor and a vaccinia virus to an individual having cancer.

15. The method of claim 14, further comprising administering an immune checkpoint inhibitor to an individual having cancer.

16. The method of claim 14, wherein the vaccinia virus and the granulopoiesis inhibitor are co-administered simultaneously, sequentially, or in reverse order.

17. The method of claim 14, wherein the granulopoiesis inhibitor is administered before, during, or after the administration of the vaccinia virus.

18. The method of claim 14, wherein the granulopoiesis inhibitor is continuously administered once daily, starting from 3 to 5 days before the administration of the vaccinia virus, and for 9 to 28 days after the administration of the vaccinia virus.

19. The method of claim 14, wherein the granulopoiesis inhibitor is administered at a dose of 10 mg/kg/day to 90 mg/kg/day.

20. The method of claim 14, wherein the vaccinia virus is administered at a dose of 1 × 10⁵ pfu to 1 × 10¹⁰ pfu.

21. The method of claim 14, wherein the vaccinia virus is administered to an individual at intervals of 7 to 30 days.

22. The method of claim 14, wherein the granulopoiesis inhibitor is administered intratumorally, intraperitoneally, or intravenously.

23. The method of claim 14, wherein the vaccinia virus is administered intratumorally, intraperitoneally, or intravenously.

24. A use of a composition comprising a vaccinia virus and a granulopoiesis inhibitor, for the prevention or treatment of cancer.

25. A use of a composition comprising a vaccinia virus and a granulopoiesis inhibitor, for the preparation of a medicament for preventing or treating cancer.

26. An anticancer adjuvant, comprising as an active ingredient:
a granulopoiesis inhibitor.

27. The anticancer adjuvant of claim 26, wherein the anticancer adjuvant is used as an anticancer adjuvant for an anticancer agent that comprises a vaccinia virus as an active ingredient.

28. The anticancer adjuvant of claim 26, wherein the anticancer adjuvant improves, enhances, or increases the anticancer activity of the vaccinia virus.

29. The anticancer adjuvant of claim 26, wherein the anticancer adjuvant increases the cancer selectivity of the vaccinia virus.

30. The anticancer adjuvant of claim 26, wherein the granulopoiesis inhibitor is hydroxyurea, lenalidomide, thalidomide, tadalafil, palbociclib, alkylating agents, anthracyclines, antimetabolites, camptothecins, epipodophyllotoxins, mitomycin C, taxanes, or vinblastine.
